(19) Europäisches Patentamt
European Patent Office
Office européen des brevets

(11) Publication number: **0 232 825 B1**

(12) # EUROPEAN PATENT SPECIFICATION

(45) Date of publication of patent specification: **13.05.92**

(51) Int. Cl.5: **C07D 237/16**, C07D 237/18, C07D 401/12, C07D 403/12, A01N 43/58

(21) Application number: **87101358.7**

(22) Date of filing: **02.02.87**

The file contains technical information submitted after the application was filed and not included in this specification

(54) **Pyridazinone derivatives, preparation thereof, and insecticidal, acaricidal, nematicidal, fungicidal compositions.**

(30) Priority: **08.02.86 JP 24978/86**

(43) Date of publication of application:
**19.08.87 Bulletin 87/34**

(45) Publication of the grant of the patent:
**13.05.92 Bulletin 92/20**

(84) Designated Contracting States:
**BE CH DE ES FR GB IT LI LU NL**

(56) References cited:
**EP-A- 0 088 384**
**EP-A- 0 134 439**
**EP-A- 0 135 076**
**EP-A- 0 183 212**
**EP-A- 0 186 817**
**EP-A- 0 193 853**

(73) Proprietor: **NISSAN CHEMICAL INDUSTRIES LTD.**
**3-7-1, Kanda Nishiki-cho**
**Chiyoda-ku Tokyo(JP)**

(72) Inventor: **Nakajima, Yasuyuki Nissan Chem.Ind.Ltd.**
**Central Research Institute 722-1, Tsuboi-cho**
**Funabashi-shi Chiba-ken(JP)**

Inventor: **Kawamura, Yasuo Nissan Chem.Ind.Ltd.**
**Central Research Institute 722-1, Tsuboi-cho**
**Funabashi-shi Chiba-ken(JP)**
Inventor: **Ogura, Tomoyuki Nissan Chem.Ind.Ltd.**
**Central Research Institute 722-1, Tsuboi-cho**
**Funabashi-shi Chiba-ken(JP)**
Inventor: **Makabe, Takahiro Nissan Chem.Ind.Ltd.**
**Central Research Institute 722-1, Tsuboi-cho**
**Funabashi-shi Chiba-ken(JP)**
Inventor: **Hirata, Kiminori Nissan Chem.Ind.Ltd.Bio.Chem.Lab.**
**1470, Ohaza Shiraoka Shiraoka-machi**
**Minamisaitama-gun Saitama-ken(JP)**
Inventor: **Kudo, Masaki Nissan Chem.Ind.Ltd.Bio.Chem.Lab.**
**1470, Ohaza Shiraoka Shiraoka-machi**
**Minamisaitama-gun Saitama-ken(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.08/2.19/2.0)

Inventor: **Ochiai, Yoshinori Nissan Chem.Ind.Ltd.Bio.Chem.Lab**
**1470, Ohaza Shiraoka Shiraoka-machi**
**Minamisaitama-gun Saitama-ken(JP)**
Inventor: **Hirose, Masayoshi Nissan Chem.Ind.Ltd.**
**3-7-1, Kanda Nishiki-cho**
**Chiyoda-ku Tokyo(JP)**

(74) Representative: **Patentanwälte Schaad, Balass & Partner**
**Dufourstrasse 101 Postfach**
**CH-8034 Zürich(CH)**

## Description

### BACKGROUND OF THE INVENTION

#### Field of the Invention

This invention relates to novel 3(2H)-pyridazinone derivatives; preparation thereof; insecticidal, acaricidal, nematicidal, fungicidal compositions for agricultural and horticultural uses; and expellent compositions for ticks parasitic on animals; said compositions containing said derivatives as an active ingredient.

#### Description of the Prior Art

The present invention concerns EP-A-0088384, EP-A-0134439, EP-A-0183212 and EP-A-0199281. The known compounds contained in these patent applications are represented by the general formula (IV):

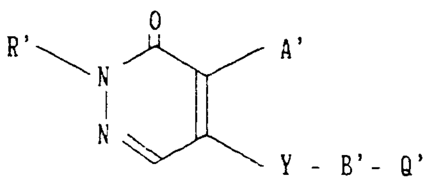

(IV)

The characteristics of the compounds of these patent applications are, e.g., in the formula (IV):
in case of EP-A-0088384 and EP-A-0134439, Y'represents oxygen atom or sulfur atom, but benzyl derivative group is bound thereto as -B'-Q'; in case of EP-A-0183212, A' represents alkyl group or it has double bond or triple bond as B';
in case of EP-A-0199281, Q represents hetrocyclic ring or specific substituent.

The present inventors have intensively conducted researches on pyridazinone derivatives which are different from these EPC patent applications in chemical structure and have obtained the present compounds of the general formula (I) given below.

Furthermore, the present inventors have found out that the present compounds of the general formula (I) given below have excellent effective insecticidal, acaricidal, nematicidal and fungicidal activities.

For example, the group of known compounds represented by the aforesaid general formula (IV) have strong insecticidal, acaricidal, nematicidal and fungicidal activities. Even in comparison with those known compounds, however, the present compounds exhibited remarkable activity rise in respect of residual activity, especially for insecticidal and acaricidal activity. Therefore, the present invention was completed by finding out that the present compounds can effectively control pests which are agriculturally and horticulturally harmful even with an extremely low drug concentration in comparison with the known compounds represented by the general formual (IV).

### SUMMARY OF THE INVENTION

An object of this invention is to provide novel 3(2H)-pyridazinone derivatives which have insecticidal, acaricidal, nematicidal and fungicidal activities.

Another object of this invention is to provide a process for preparing such 3(2H)-pyridazinone derivatives.

Further object of this invention is to provide insecticidal acaricidal, nematicidal, fungicidal compositions containing a 3(2H)-pyridazinone derivatives as an active ingredient.

Still further object of this invention is to provide a method for controlling pests by using the above-mentioned derivatives or compositions.

Other objects of this invention will become apparent from the description given below.

Detailed Description of the Invention

The pyridazinone derivatives according to the invention have the general formula (I):

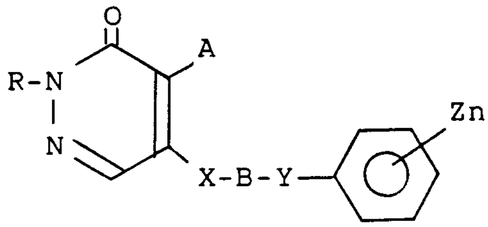

(I)

wherein

R represents tertiary butyl,

A represents chlorine atom,

B represents $-CH_2-CH_2-$,

X represents oxygen atom or sulfur atom,

Y represents oxygen atom,

n is an integer of 1 to 5 and when n is 2 to 5, Z may be same or different,

Z represents halogen atom, straight or branched chain alkyl group having 1 to 10 carbon atoms, straight or branched chain alkoxy group having 1 to 10 carbon atoms, alkylcarbonyl having 2 to 10 carbon atoms,

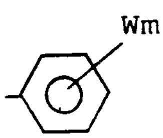 , 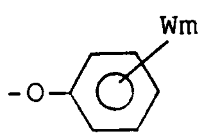 , 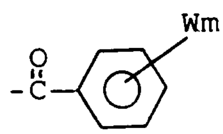 or 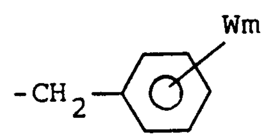

W represents halogen atom, alkyl group having 1 to 4 carbon atoms, alkoxy group having 1 to 4 carbon atoms or nitro group,

m is 0 or an integer of 1 to 2, and when m is 2, W may be same or different.

In respect to the activity for controlling pests, preferable compounds of the present invention are ones in which, in the general formula (I), Z represents halogen atom or alkyl group having 1 to 10 carbon atoms in 2 position and 6 position and represents one of alkyl group having 1 to 10 carbon atoms, alkoxy group having 1 to 10 carbon atoms, alkylcarbonyl group having 2 to 10 carbon atoms,

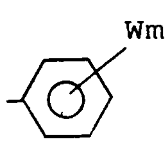 , 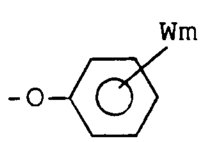 , 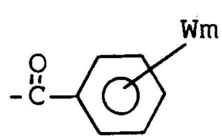 or 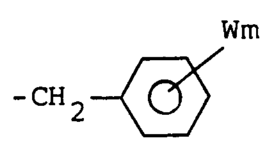 .

More especially preferred compounds of the general formula (I) among the compounds shown in Tables 1 to 2 are:

Nos. 36, 38, 40, 46, 49, 55, 71, 72, 73, 74, 84, 85, 88, 89, 92, 111, 112, 113, 463, 465, 466, 468, 470, 472, 474, 476, 477, 480, 482, 484, 490, 491, 492, 494, 496, 514, 517, 518, 520 and 542.

As the compounds covered by the present invention, for example, the compounds shown in Tables 1, 2 are exemplified. However, the present invention is not restricted to these compounds which are shown as only exemplification.

In Tables 1, 2 t, i, c and s mean tertiary, iso, cyclo and secondary, respectively, and each symbol Me, Et, Pr, Bu, Am, Pen, Hex and Ph means methyl, ethyl, propyl, butyl, amyl, pentyl, hexyl and phenyl, respectively.

In case of the compounds containing asymmetric carbon atoms among the compounds covered by the present invention, optical isomers, i.e., (+)-isomers and (-)-isomers are also included in the present invention.

Table 1

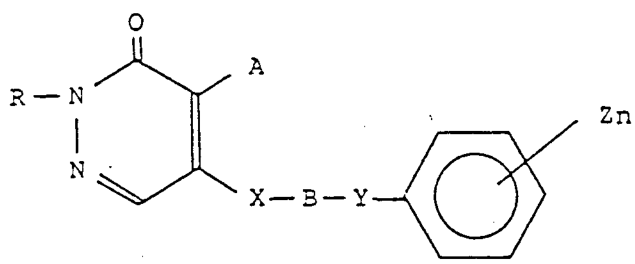

| No. | R | A | X | B | Y | Zn |
|---|---|---|---|---|---|---|
| 12 | t-Bu | Cl | S | $CH_2CH_2$ | O | 2-Me |
| 13 | t-Bu | Cl | S | $CH_2CH_2$ | O | 4-Et |
| 14 | t-Bu | Cl | S | $CH_2CH_2$ | O | 4-Pr |
| 15 | t-Bu | Cl | S | $CH_2CH_2$ | O | 2-i-Pr |
| 16 | t-Bu | Cl | S | $CH_2CH_2$ | O | 4-Bu |
| 17 | t-Bu | Cl | S | $CH_2CH_2$ | O | 4-Pen |
| 18 | t-Bu | Cl | S | $CH_2CH_2$ | O | 3-OPr |
| 19 | t-Bu | Cl | S | $CH_2CH_2$ | O | 4-OBu |
| 21 | t-Bu | Cl | S | $CH_2CH_2$ | O | 2-Me, 4-Cl |
| 22 | t-Bu | Cl | S | $CH_2CH_2$ | O | 2-Me, 4-Me |
| 23 | t-Bu | Cl | S | $CH_2CH_2$ | O | 2-Me, 5-Me |
| 24 | t-Bu | Cl | S | $CH_2CH_2$ | O | 2-Me, 6-Me |
| 25 | t-Bu | Cl | S | $CH_2CH_2$ | O | 2-Me, 4-Pen |
| 26 | t-Bu | Cl | S | $CH_2CH_2$ | O | 2-Et, 4-Pen |
| 27 | t-Bu | Cl | S | $CH_2CH_2$ | O | 2-Me, 4-CO$(CH_2)_3CH_3$ |
| 28 | t-Bu | Cl | S | $CH_2CH_2$ | O | 2-OMe, 6-OMe |
| 29 | t-Bu | Cl | S | $CH_2CH_2$ | O | 2-Me, 6-Cl |
| 30 | t-Bu | Cl | S | $CH_2CH_2$ | O | 2-Cl, 6-Cl |

| No. | R | A | X | B | Y | Zn |
|---|---|---|---|---|---|---|
| 31 | t-Bu | Cl | S | $CH_2CH_2$ | 0 | 2-Me, 4-Cl, 6-Cl |
| 32 | t-Bu | Cl | S | $CH_2CH_2$ | 0 | 2-Me, 3-Me, 6-Me |
| 33 | t-Bu | Cl | S | $CH_2CH_2$ | 0 | 2-Me, 4-Me, 6-Me |
| 34 | t-Bu | Cl | S | $CH_2CH_2$ | 0 | 2-Me, 4-Br, 6-Cl |
| 35 | t-Bu | Cl | S | $CH_2CH_2$ | 0 | 2-Me, 4-$COCH_2CH_3$, 6-Cl |
| 36 | t-Bu | Cl | S | $CH_2CH_2$ | 0 | 2-Me, 4-Pr, 6-Cl |
| 37 | t-Bu | Cl | S | $CH_2CH_2$ | 0 | 2-Me, 4-Bu, 6-Cl |
| 38 | t-Bu | Cl | S | $CH_2CH_2$ | 0 | 2-Cl, 4-$COCH_2CH_3$, 6-Cl |
| 39 | t-Bu | Cl | S | $CH_2CH_2$ | 0 | 2-Cl, 4-Et, 6-Cl |
| 40 | t-Bu | Cl | S | $CH_2CH_2$ | 0 | 2-Cl, 4-Pr, 6-Cl |
| 41 | t-Bu | Cl | S | $CH_2CH_2$ | 0 | 2-Cl, 4-i-Pr, 6-Cl |
| 42 | t-Bu | Cl | S | $CH_2CH_2$ | 0 | 2-Cl, 4-Bu, 6-Cl |
| 43 | t-Bu | Cl | S | $CH_2CH_2$ | 0 | 2-Cl, 4-i-Bu, 6-Cl |
| 44 | t-Bu | Cl | S | $CH_2CH_2$ | 0 | 2-Cl, 4-t-Bu, 6-Cl |
| 45 | t-Bu | Cl | S | $CH_2CH_2$ | 0 | 2-Cl, 4-Pen, 6-Cl |
| 46 | t-Bu | Cl | S | $CH_2CH_2$ | 0 | 2-Cl, 4-Ph, 6-Cl |
| 47 | t-Bu | Cl | S | $CH_2CH_2$ | 0 | 2-Br, 4-$COCH_2CH_3$, 6-Br |
| 48 | t-Bu | Cl | S | $CH_2CH_2$ | 0 | 2-Br, 4-Et, 6-Br |
| 49 | t-Bu | Cl | S | $CH_2CH_2$ | 0 | 2-Br, 4-Pr, 6-Br |
| 50 | t-Bu | Cl | S | $CH_2CH_2$ | 0 | 2-Br, 4-i-Pr, 6-Br |
| 51 | t-Bu | Cl | S | $CH_2CH_2$ | 0 | 2-Br, 4-Bu, 6-Br |
| 52 | t-Bu | Cl | S | $CH_2CH_2$ | 0 | 2-Br, 4-i-Bu, 6-Br |
| 53 | t-Bu | Cl | S | $CH_2CH_2$ | 0 | 2-Br, 4-t-Bu, 6-Br |
| 54 | t-Bu | Cl | S | $CH_2CH_2$ | 0 | 2-Br, 4-Ph, 6-Br |
| 55 | t-Bu | Cl | S | $CH_2CH_2$ | 0 | 2-F, 4-$COCH_2CH_3$, 6-F |
| 57 | t-Bu | Cl | S | $CH_2CH_2$ | 0 | 2-F, 4-Pr, 6-F |
| 58 | t-Bu | Cl | S | $CH_2CH_2$ | 0 | 2-F, 4-Bu, 6-F |
| 59 | t-Bu | Cl | S | $CH_2CH_2$ | 0 | 2-F, 4-Pen, 6-F |
| 60 | t-Bu | Cl | S | $CH_2CH_2$ | 0 | 2-F, 4-Et, 6-F |

| No. | R | A | X | B | Y | Zn |
|---|---|---|---|---|---|---|
| 66 | t-Bu | Cl | S | $CH_2CH_2$ | O | 2-OMe, 4-Et, 6-OMe |
| 67 | t-Bu | Cl | S | $CH_2CH_2$ | O | 2-OMe, 4-Pr, 6-OMe |
| 68 | t-Bu | Cl | S | $CH_2CH_2$ | O | 2-OMe, 4-i-Pr, 6-OMe |
| 69 | t-Bu | Cl | S | $CH_2CH_2$ | O | 2-OMe, 4-Bu, 6-OMe |
| 70 | t-Bu | Cl | S | $CH_2CH_2$ | O | 2-OMe, 4-t-Bu, 6-OMe |
| 71 | t-Bu | Cl | S | $CH_2CH_2$ | O | 2,6-$Me_2$, 4-$COCH_3$ |
| 72 | t-Bu | Cl | S | $CH_2CH_2$ | O | 2,6-$Me_2$, 4-$COCH_2CH_3$ |
| 73 | t-Bu | Cl | S | $CH_2CH_2$ | O | 2,6-$Me_2$, 4-$COCH_2CH_2CH_3$ |
| 74 | t-Bu | Cl | S | $CH_2CH_2$ | O | 2,6-$Me_2$, 4-$COCH(CH_3)_2$ |
| 76 | t-Bu | Cl | S | $CH_2CH_2$ | O | 2,6-$Me_2$, 4-$CO(CH_2)_3CH_3$ |
| 78 | t-Bu | Cl | S | $CH_2CH_2$ | O | 2,6-$Me_2$, 4-$CO(CH_2)_4CH_3$ |
| 79 | t-Bu | Cl | S | $CH_2CH_2$ | O | 2,6-$Me_2$, 4-$CO(CH_2)_6CH_3$ |
| 80 | t-Bu | Cl | S | $CH_2CH_2$ | O | 2,6-$Me_2$, 4-$CO(CH_2)_8CH_3$ |
| 81 | t-Bu | Cl | S | $CH_2CH_2$ | O | 2,6-$Me_2$, 4-Cl |
| 82 | t-Bu | Cl | S | $CH_2CH_2$ | O | 2,6-$Me_2$, 4-Br |
| 83 | t-Bu | Cl | S | $CH_2CH_2$ | O | 2,6-$Me_2$, 4-I |
| 84 | t-Bu | Cl | S | $CH_2CH_2$ | O | 2,6-$Me_2$, 4-Et |
| 85 | t-Bu | Cl | S | $CH_2CH_2$ | O | 2,6-$Me_2$, 4-Pr |
| 86 | t-Bu | Cl | S | $CH_2CH_2$ | O | 2,6-$Me_2$, 4-i-Pr |
| 88 | t-Bu | Cl | S | $CH_2CH_2$ | O | 2,6-$Me_2$, 4-Bu |
| 89 | t-Bu | Cl | S | $CH_2CH_2$ | O | 2,6-$Me_2$, 4-i-Bu |

| No. | R | A | X | B | Y | Zn |
|---|---|---|---|---|---|---|
| 92 | t-Bu | Cl | S | $CH_2CH_2$ | 0 | 2,6-$Me_2$,4-t-Bu |
| 93 | t-Bu | Cl | S | $CH_2CH_2$ | 0 | 2,6-$Me_2$,4-Pen |
| 94 | t-Bu | Cl | S | $CH_2CH_2$ | 0 | 2,6-$Me_2$,4-t-Am |
| 95 | t-Bu | Cl | S | $CH_2CH_2$ | 0 | 2,6-$Me_2$,4-Hex |
| 97 | t-Bu | Cl | S | $CH_2CH_2$ | 0 | 2,6-$Me_2$,4-$(CH_2)_7CH_3$ |
| 98 | t-Bu | Cl | S | $CH_2CH_2$ | 0 | 2,6-$Me_2$,4-$(CH_2)_9CH_3$ |
| 100 | t-Bu | Cl | S | $CH_2CH_2$ | 0 | 2,6-$Me_2$,4-Ph |
| 111 | t-Bu | Cl | S | $CH_2CH_2$ | 0 | 2,6-$Me_2$,4-OMe |
| 112 | t-Bu | Cl | S | $CH_2CH_2$ | 0 | 2,6-$Me_2$,4-OEt |
| 113 | t-Bu | Cl | S | $CH_2CH_2$ | 0 | 2,6-$Me_2$,4-OPr |
| 114 | t-Bu | Cl | S | $CH_2CH_2$ | 0 | 2,6-$Me_2$,4-O-i-Pr |
| 115 | t-Bu | Cl | S | $CH_2CH_2$ | 0 | 2,6-$Me_2$,4-OBu |
| 116 | t-Bu | Cl | S | $CH_2CH_2$ | 0 | 2,6-$Me_2$,4-O-t-Bu |
| 117 | t-Bu | Cl | S | $CH_2CH_2$ | 0 | 2,6-$Me_2$,4-OPen |

| No. | R | A | X | B | Y | Zn |
|---|---|---|---|---|---|---|
| 148 | t-Bu | Cl | S | $CH_2CH_2$ | 0 | 2,4,6-$Cl_3$,3-Et |
| 149 | t-Bu | Cl | S | $CH_2CH_2$ | 0 | 2,4,6-$Cl_3$,3-Pr |
| 150 | t-Bu | Cl | S | $CH_2CH_2$ | 0 | 2,4,6-$Cl_3$,3-t-Bu |

| No. | R | A | X | B | Y | Zn |
|---|---|---|---|---|---|---|
| 202 | t-Bu | Cl | O | $CH_2CH_2$ | 0 | 2-Me |
| 203 | t-Bu | Cl | O | $CH_2CH_2$ | 0 | 4-Et |
| 204 | t-Bu | Cl | O | $CH_2CH_2$ | 0 | 4-Pr |
| 205 | t-Bu | Cl | O | $CH_2CH_2$ | 0 | 2-i-Pr |
| 206 | t-Bu | Cl | O | $CH_2CH_2$ | 0 | 4-Bu |
| 207 | t-Bu | Cl | O | $CH_2CH_2$ | 0 | 4-t-Bu |
| 208 | t-Bu | Cl | O | $CH_2CH_2$ | 0 | 4-Pen |
| 210 | t-Bu | Cl | O | $CH_2CH_2$ | 0 | 4-Ph |

| No. | R | A | X | B | Y | Zn |
|---|---|---|---|---|---|---|
| 212 | t-Bu | Cl | O | $CH_2CH_2$ | O | 3-OPr |
| 213 | t-Bu | Cl | O | $CH_2CH_2$ | O | 4-OBu |
| 215 | t-Bu | Cl | O | $CH_2CH_2$ | O | 2-Me, 4-Cl |
| 216 | t-Bu | Cl | O | $CH_2CH_2$ | O | 2-Me, 3-Me |
| 217 | t-Bu | Cl | O | $CH_2CH_2$ | O | 2-Me, 4-Me |
| 218 | t-Bu | Cl | O | $CH_2CH_2$ | O | 2-Me, 5-Me |
| 219 | t-Bu | Cl | O | $CH_2CH_2$ | O | 2-Me, 6-Me |
| 220 | t-Bu | Cl | O | $CH_2CH_2$ | O | 2-Me, 4-Pen |
| 221 | t-Bu | Cl | O | $CH_2CH_2$ | O | 2-Et, 4-Pen |
| 222 | t-Bu | Cl | O | $CH_2CH_2$ | O | 2-Me, 4-$CO(CH_2)_3CH_3$ |
| 223 | t-Bu | Cl | O | $CH_2CH_2$ | O | 2-OMe, 6-OMe |
| 224 | t-Bu | Cl | O | $CH_2CH_2$ | O | 2-Me, 6-Cl |
| 225 | t-Bu | Cl | O | $CH_2CH_2$ | O | 2-Cl, 6-Cl, |
| 226 | t-Bu | Cl | O | $CH_2CH_2$ | O | 2-Me, 4-Cl, 6-Cl |
| 228 | t-Bu | Cl | O | $CH_2CH_2$ | O | 2-Me, 3-Me, 6-Me |
| 229 | t-Bu | Cl | O | $CH_2CH_2$ | O | 2-Me, 4-Me, 6-Me |
| 230 | t-Bu | Cl | O | $CH_2CH_2$ | O | 2-Me, 4-Br, 6-Cl |
| 231 | t-Bu | Cl | O | $CH_2CH_2$ | O | 2-Me, 4-$COCH_2CH_3$, 6-Cl |
| 232 | t-Bu | Cl | O | $CH_2CH_2$ | O | 2-Me, 4-Pr, 6-Cl |
| 233 | t-Bu | Cl | O | $CH_2CH_2$ | O | 2-Cl, 4-$COCH_2CH_3$, 6-Cl |
| 234 | t-Bu | Cl | O | $CH_2CH_2$ | O | 2-Cl, 4-Et, 6-Cl |
| 235 | t-Bu | Cl | O | $CH_2CH_2$ | O | 2-Cl, 4-Pr, 6-Cl |
| 236 | t-Bu | Cl | O | $CH_2CH_2$ | O | 2-Cl, 4-i-Pr, 6-Cl |
| 237 | t-Bu | Cl | O | $CH_2CH_2$ | O | 2-Cl, 4-Bu, 6-Cl |
| 238 | t-Bu | Cl | O | $CH_2CH_2$ | O | 2-Cl, 4-i-Bu, 6-Cl |
| 239 | t-Bu | Cl | O | $CH_2CH_2$ | O | 2-Cl, 4-t-Bu, 6-Cl |
| 240 | t-Bu | Cl | O | $CH_2CH_2$ | O | 2-Cl, 4-Pen, 6-Cl |

| No. | R | A | X | B | Y | Zn |
|---|---|---|---|---|---|---|
| 241 | t-Bu | Cl | O | $CH_2CH_2$ | O | 2-Cl,4-Ph,6-Cl |
| 242 | t-Bu | Cl | O | $CH_2CH_2$ | O | 2-Br,4-$COCH_2CH_3$,6-Br |
| 243 | t-Bu | Cl | O | $CH_2CH_2$ | O | 2-Br,4-Et,6-Br |
| 244 | t-Bu | Cl | O | $CH_2CH_2$ | O | 2-Br,4-Pr,6-Br |
| 245 | t-Bu | Cl | O | $CH_2CH_2$ | O | 2-Br,4-i-Pr,6-Br |
| 246 | t-Bu | Cl | O | $CH_2CH_2$ | O | 2-Br,4-Bu,6-Br |
| 247 | t-Bu | Cl | O | $CH_2CH_2$ | O | 2-Br,4-i-Bu,6-Br |
| 248 | t-Bu | Cl | O | $CH_2CH_2$ | O | 2-Br,4-t-Bu,6-Br |
| 249 | t-Bu | Cl | O | $CH_2CH_2$ | O | 2-Br,4-Ph,6-Br |
| 250 | t-Bu | Cl | O | $CH_2CH_2$ | O | 2-F,4-$COCH_2CH_3$,6-F |
| 252 | t-Bu | Cl | O | $CH_2CH_2$ | O | 2-F,4-Pr,6-F |
| 253 | t-Bu | Cl | O | $CH_2CH_2$ | O | 2-F,4-Bu,6-F |
| 254 | t-Bu | Cl | O | $CH_2CH_2$ | O | 2-F,4-Pen,6-F |
| 255 | t-Bu | Cl | O | $CH_2CH_2$ | O | 2-F,4-Et,6-F |
| 261 | t-Bu | Cl | O | $CH_2CH_2$ | O | 2-OMe,4-Et,6-OMe |
| 262 | t-Bu | Cl | O | $CH_2CH_2$ | O | 2-OMe,4-Pr,6-OMe |
| 263 | t-Bu | Cl | O | $CH_2CH_2$ | O | 2-OMe,4-i-Pr,6-OMe |
| 264 | t-Bu | Cl | O | $CH_2CH_2$ | O | 2-OMe,4-Bu,6-OMe |
| 265 | t-Bu | Cl | O | $CH_2CH_2$ | O | 2-OMe,4-t-Bu,6-OMe |
| 266 | t-Bu | Cl | O | $CH_2CH_2$ | O | 2,6-$Me_2$,4-$COCH_3$ |
| 267 | t-Bu | Cl | O | $CH_2CH_2$ | O | 2,6-$Me_2$,4-$COCH_2CH_3$ |
| 268 | t-Bu | Cl | O | $CH_2CH_2$ | O | 2,6-$Me_2$,4-$COCH_2CH_2CH_3$ |
| 269 | t-Bu | Cl | O | $CH_2CH_2$ | O | 2,6-$Me_2$,4-$COCH(CH_3)_2$ |

| No. | R | A | X | B | Y | Zn |
|---|---|---|---|---|---|---|
| 271 | t-Bu | Cl | O | $CH_2CH_2$ | O | 2,6-$Me_2$,4-$CO(CH_2)_3CH_3$ |
| 273 | t-Bu | Cl | O | $CH_2CH_2$ | O | 2,6-$Me_2$,4-$CO(CH_2)_4CH_3$ |
| 274 | t-Bu | Cl | O | $CH_2CH_2$ | O | 2,6-$Me_2$,4-$CO(CH_2)_6CH_3$ |
| 275 | t-Bu | Cl | O | $CH_2CH_2$ | O | 2,6-$Me_2$,4-$CO(CH_2)_8CH_3$ |
| 276 | t-Bu | Cl | O | $CH_2CH_2$ | O | 2,6-$Me_2$,4-Cl |
| 277 | t-Bu | Cl | O | $CH_2CH_2$ | O | 2,6-$Me_2$,4-Br |
| 278 | t-Bu | Cl | O | $CH_2CH_2$ | O | 2,6-$Me_2$,4-I |
| 279 | t-Bu | Cl | O | $CH_2CH_2$ | O | 2,6-$Me_2$,4-Et |
| 280 | t-Bu | Cl | O | $CH_2CH_2$ | O | 2,6-$Me_2$,4-Pr |
| 281 | t-Bu | Cl | O | $CH_2CH_2$ | O | 2,6-$Me_2$,4-i-Pr |
| 283 | t-Bu | Cl | O | $CH_2CH_2$ | O | 2,6-$Me_2$,4-Bu |
| 284 | t-Bu | Cl | O | $CH_2CH_2$ | O | 2,6-$Me_2$,4-i-Bu |
| 287 | t-Bu | Cl | O | $CH_2CH_2$ | O | 2,6-$Me_2$,4-t-Bu |
| 288 | t-Bu | Cl | O | $CH_2CH_2$ | O | 2,6-$Me_2$,4-Pen |
| 289 | t-Bu | Cl | O | $CH_2CH_2$ | O | 2,6-$Me_2$,4-t-Am |
| 290 | t-Bu | Cl | O | $CH_2CH_2$ | O | 2,6-$Me_2$,4-Hex |
| 292 | t-Bu | Cl | O | $CH_2CH_2$ | O | 2,6-$Me_2$,4-$(CH_2)_7CH_3$ |
| 293 | t-Bu | Cl | O | $CH_2CH_2$ | O | 2,6-$Me_2$,4-$(CH_2)_9CH_3$ |
| 295 | t-Bu | Cl | O | $CH_2CH_2$ | O | 2,6-$Me_2$,4-Ph |

| No. | R | A | X | B | Y | Zn |
|---|---|---|---|---|---|---|
| 306 | t-Bu | Cl | O | $CH_2CH_2$ | O | 2,6-$Me_2$,4-OMe |
| 307 | t-Bu | Cl | O | $CH_2CH_2$ | O | 2,6-$Me_2$,4-OEt |
| 308 | t-Bu | Cl | O | $CH_2CH_2$ | O | 2,6-$Me_2$,4-OPr |
| 309 | t-Bu | Cl | O | $CH_2CH_2$ | O | 2,6-$Me_2$,4-O-i-Pr |
| 310 | t-Bu | Cl | O | $CH_2CH_2$ | O | 2,6-$Me_2$,4-OBu |
| 311 | t-Bu | Cl | O | $CH_2CH_2$ | O | 2,6-$Me_2$,4-O-t-Bu |
| 312 | t-Bu | Cl | O | $CH_2CH_2$ | O | 2,6-$Me_2$,4-OPen |

| No. | R | A | X | B | Y | Zn |
|---|---|---|---|---|---|---|
| 343 | t-Bu | Cl | O | $CH_2CH_2$ | O | 2,4,6-$Cl_3$,3-Et |
| 344 | t-Bu | Cl | O | $CH_2CH_2$ | O | 2,4,6-$Cl_3$,3-t-Bu |

Table 2

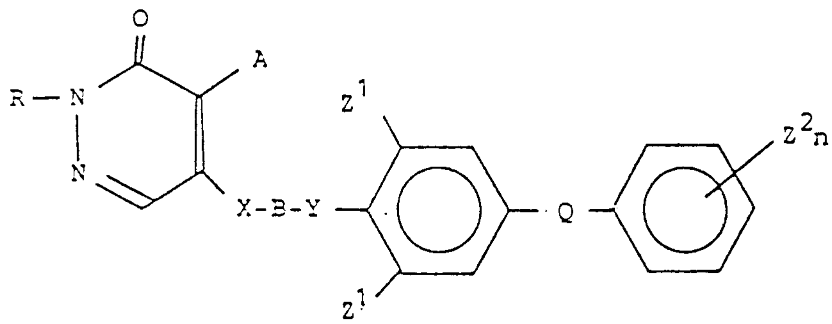

| No. | R | A | X | B | Y | $Z^1$ | Q | $Z^2n$ |
|---|---|---|---|---|---|---|---|---|
| 421 | t-Bu | Cl | S | $CH_2CH_2$ | O | H | O | H |
| 422 | t-Bu | Cl | S | $CH_2CH_2$ | O | H | O | 4-Cl |
| 423 | t-Bu | Cl | S | $CH_2CH_2$ | O | H | O | 4-Me |
| 424 | t-Bu | Cl | S | $CH_2CH_2$ | O | Cl | O | H |
| 425 | t-Bu | Cl | S | $CH_2CH_2$ | O | Cl | O | 4-Me |
| 426 | t-Bu | Cl | S | $CH_2CH_2$ | O | Me | O | H |
| 427 | t-Bu | Cl | S | $CH_2CH_2$ | O | Me | O | 4-Cl |
| 428 | t-Bu | Cl | S | $CH_2CH_2$ | O | Me | O | 2-Me |
| 429 | t-Bu | Cl | S | $CH_2CH_2$ | O | Me | O | 4-Me |
| 430 | t-Bu | Cl | S | $CH_2CH_2$ | O | Me | O | 4-Et |
| 431 | t-Bu | Cl | S | $CH_2CH_2$ | O | Me | O | 4-t-Bu |
| 432 | t-Bu | Cl | S | $CH_2CH_2$ | O | Me | O | 4-OMe |
| 434 | t-Bu | Cl | S | $CH_2CH_2$ | O | Me | O | 4-$NO_2$ |
| 437 | t-Bu | Cl | S | $CH_2CH_2$ | O | Me | O | 2-Cl,4-Cl |
| 438 | t-Bu | Cl | S | $CH_2CH_2$ | O | Me | O | 2-Me,4-Me |

| No. | R | A | X | B | Y | $Z^1$ | Q | $Z^2n$ |
|---|---|---|---|---|---|---|---|---|
| 459 | t-Bu | Cl | S | $CH_2CH_2$ | O | H | CO | H |
| 460 | t-Bu | Cl | S | $CH_2CH_2$ | O | H | CO | 4-Cl |
| 461 | t-Bu | Cl | S | $CH_2CH_2$ | O | H | CO | 4-Me |
| 462 | t-Bu | Cl | S | $CH_2CH_2$ | O | Cl | CO | H |
| 463 | t-Bu | Cl | S | $CH_2CH_2$ | O | Cl | CO | 4-Cl |
| 464 | t-Bu | Cl | S | $CH_2CH_2$ | O | Cl | CO | 4-Me |
| 465 | t-Bu | Cl | S | $CH_2CH_2$ | O | Me | CO | H |
| 466 | t-Bu | Cl | S | $CH_2CH_2$ | O | Me | CO | 2-Cl |
| 467 | t-Bu | Cl | S | $CH_2CH_2$ | O | Me | CO | 3-Cl |
| 468 | t-Bu | Cl | S | $CH_2CH_2$ | O | Me | CO | 4-Cl |
| 469 | t-Bu | Cl | S | $CH_2CH_2$ | O | Me | CO | 2-F |
| 470 | t-Bu | Cl | S | $CH_2CH_2$ | O | Me | CO | 4-F |
| 471 | t-Bu | Cl | S | $CH_2CH_2$ | O | Me | CO | 2-Br |
| 472 | t-Bu | Cl | S | $CH_2CH_2$ | O | Me | CO | 4-Br |
| 473 | t-Bu | Cl | S | $CH_2CH_2$ | O | Me | CO | 4-I |
| 474 | t-Bu | Cl | S | $CH_2CH_2$ | O | Me | CO | 2-Me |
| 475 | t-Bu | Cl | S | $CH_2CH_2$ | O | Me | CO | 3-Me |
| 476 | t-Bu | Cl | S | $CH_2CH_2$ | O | Me | CO | 4-Me |
| 477 | t-Bu | Cl | S | $CH_2CH_2$ | O | Me | CO | 4-Et |
| 478 | t-Bu | Cl | S | $CH_2CH_2$ | O | Me | CO | 4-Bu |
| 479 | t-Bu | Cl | S | $CH_2CH_2$ | O | Me | CO | 4-i-Pr |
| 480 | t-Bu | Cl | S | $CH_2CH_2$ | O | Me | CO | 4-t-Bu |

| No. | R | A | X | B | Y | $Z^1$ | Q | $Z^2_n$ |
|---|---|---|---|---|---|---|---|---|
| 482 | t-Bu | Cl | S | $CH_2CH_2$ | O | Me | CO | 4-$NO_2$ |
| 484 | t-Bu | Cl | S | $CH_2CH_2$ | O | Me | CO | 4-OMe |
| 485 | t-Bu | Cl | S | $CH_2CH_2$ | O | Me | CO | 4-OEt |
| 488 | t-Bu | Cl | S | $CH_2CH_2$ | O | Me | CO | 2-F, 4-F |
| 489 | t-Bu | Cl | S | $CH_2CH_2$ | O | Me | CO | 2-F, 6-F |
| 490 | t-Bu | Cl | S | $CH_2CH_2$ | O | Me | CO | 2-Cl, 4-Cl |
| 491 | t-Bu | Cl | S | $CH_2CH_2$ | O | Me | CO | 2-Cl, 6-Cl |
| 492 | t-Bu | Cl | S | $CH_2CH_2$ | O | Me | CO | 3-Cl, 4-Cl |
| 493 | t-Bu | Cl | S | $CH_2CH_2$ | O | Me | CO | 3-Cl, 5-Cl |
| 494 | t-Bu | Cl | S | $CH_2CH_2$ | O | Me | CO | 2-Cl, 4-$NO_2$ |
| 495 | t-Bu | Cl | S | $CH_2CH_2$ | O | Me | CO | 2-$NO_2$, 4-Cl |
| 496 | t-Bu | Cl | S | $CH_2CH_2$ | O | Me | CO | 2-Me, 4-Me |
| 497 | t-Bu | Cl | S | $CH_2CH_2$ | O | Me | CO | 2-Me, 6-Me |
| 498 | t-Bu | Cl | S | $CH_2CH_2$ | O | Me | CO | 3-Me, 4-Me |
| 499 | t-Bu | Cl | S | $CH_2CH_2$ | O | Me | CO | 3-Me, 5-Me |
| 501 | t-Bu | Cl | S | $CH_2CH_2$ | O | Me | CO | 2-Cl, 4-Me |
| 502 | t-Bu | Cl | S | $CH_2CH_2$ | O | Me | CO | 2-Cl, 6-Me |
| 503 | t-Bu | Cl | S | $CH_2CH_2$ | O | Me | CO | 2-Me, 4-Cl |
| 510 | t-Bu | Cl | S | $CH_2CH_2$ | O | i-Pr | CO | H |

| No. | R | A | X | B | Y | $Z^1$ | Q | $Z^2n$ |
|---|---|---|---|---|---|---|---|---|
| 511 | t-Bu | Cl | S | $CH_2CH_2$ | O | H | $CH_2$ | H |
| 512 | t-Bu | Cl | S | $CH_2CH_2$ | O | H | $CH_2$ | 4-Cl |
| 513 | t-Bu | Cl | S | $CH_2CH_2$ | O | H | $CH_2$ | 4-Me |
| 514 | t-Bu | Cl | S | $CH_2CH_2$ | O | Cl | $CH_2$ | H |
| 515 | t-Bu | Cl | S | $CH_2CH_2$ | O | Cl | $CH_2$ | 4-Cl |
| 516 | t-Bu | Cl | S | $CH_2CH_2$ | O | Cl | $CH_2$ | 4-Me |
| 517 | t-Bu | Cl | S | $CH_2CH_2$ | O | Me | $CH_2$ | H |
| 518 | t-Bu | Cl | S | $CH_2CH_2$ | O | Me | $CH_2$ | 2-Cl |
| 519 | t-Bu | Cl | S | $CH_2CH_2$ | O | Me | $CH_2$ | 3-Cl |
| 520 | t-Bu | Cl | S | $CH_2CH_2$ | O | Me | $CH_2$ | 4-Cl |
| 521 | t-Bu | Cl | S | $CH_2CH_2$ | O | Me | $CH_2$ | 2-F |
| 522 | t-Bu | Cl | S | $CH_2CH_2$ | O | Me | $CH_2$ | 4-F |
| 523 | t-Bu | Cl | S | $CH_2CH_2$ | O | Me | $CH_2$ | 2-Br |
| 524 | t-Bu | Cl | S | $CH_2CH_2$ | O | Me | $CH_2$ | 4-Br |
| 525 | t-Bu | Cl | S | $CH_2CH_2$ | O | Me | $CH_2$ | 4-I |
| 526 | t-Bu | Cl | S | $CH_2CH_2$ | O | Me | $CH_2$ | 2-Me |
| 527 | t-Bu | Cl | S | $CH_2CH_2$ | O | Me | $CH_2$ | 3-Me |
| 528 | t-Bu | Cl | S | $CH_2CH_2$ | O | Me | $CH_2$ | 4-Me |
| 529 | t-Bu | Cl | S | $CH_2CH_2$ | O | Me | $CH_2$ | 4-Et |
| 530 | t-Bu | Cl | S | $CH_2CH_2$ | O | Me | $CH_2$ | 4-Bu |
| 531 | t-Bu | Cl | S | $CH_2CH_2$ | O | Me | $CH_2$ | 4-i-Pr |
| 532 | t-Bu | Cl | S | $CH_2CH_2$ | O | Me | $CH_2$ | 4-t-Bu |
| 534 | t-Bu | Cl | S | $CH_2CH_2$ | O | Me | $CH_2$ | 4-$NO_2$ |
| 536 | t-Bu | Cl | S | $CH_2CH_2$ | O | Me | $CH_2$ | 4-OMe |
| 537 | t-Bu | Cl | S | $CH_2CH_2$ | O | Me | $CH_2$ | 4-OEt |
| 540 | t-Bu | Cl | S | $CH_2CH_2$ | O | Me | $CH_2$ | 2-F,4-F |

| No. | R | A | X | B | Y | $Z^1$ | Q | $Z^2n$ |
|---|---|---|---|---|---|---|---|---|
| 541 | t-Bu | Cl | S | $CH_2CH_2$ | O | Me | $CH_2$ | 2-F,6-F |
| 542 | t-Bu | Cl | S | $CH_2CH_2$ | O | Me | $CH_2$ | 2-Cl,4-Cl |
| 543 | t-Bu | Cl | S | $CH_2CH_2$ | O | Me | $CH_2$ | 2-Cl,6-Cl |
| 544 | t-Bu | Cl | S | $CH_2CH_2$ | O | Me | $CH_2$ | 3-Cl,4-Cl |
| 545 | t-Bu | Cl | S | $CH_2CH_2$ | O | Me | $CH_2$ | 3-Cl,5-Cl |
| 546 | t-Bu | Cl | S | $CH_2CH_2$ | O | Me | $CH_2$ | 2-Cl,4-$NO_2$ |
| 547 | t-Bu | Cl | S | $CH_2CH_2$ | O | Me | $CH_2$ | 2-$NO_2$,4-Cl |
| 548 | t-Bu | Cl | S | $CH_2CH_2$ | O | Me | $CH_2$ | 2-Me,4-Me |
| 549 | t-Bu | Cl | S | $CH_2CH_2$ | O | Me | $CH_2$ | 2-Me,6-Me |
| 550 | t-Bu | Cl | S | $CH_2CH_2$ | O | Me | $CH_2$ | 3-Me,4-Me |
| 551 | t-Bu | Cl | S | $CH_2CH_2$ | O | Me | $CH_2$ | 3-Me,5-Me |
| 553 | t-Bu | Cl | S | $CH_2CH_2$ | O | Me | $CH_2$ | 2-Cl,4-Me |
| 554 | t-Bu | Cl | S | $CH_2CH_2$ | O | Me | $CH_2$ | 2-Cl,6-Me |
| 555 | t-Bu | Cl | S | $CH_2CH_2$ | O | Me | $CH_2$ | 2-Me,4-Cl |
| 562 | t-Bu | Cl | S | $CH_2CH_2$ | O | i-Pr | $CH_2$ | H |

| No. | R | A | X | B | Y | $Z^1$ | Q | $Z^2n$ |
|---|---|---|---|---|---|---|---|---|
| 601 | t-Bu | Cl | O | $CH_2CH_2$ | O | H | O | H |
| 602 | t-Bu | Cl | O | $CH_2CH_2$ | O | H | O | 4-Cl |
| 603 | t-Bu | Cl | O | $CH_2CH_2$ | O | H | O | 4-Me |
| 604 | t-Bu | Cl | O | $CH_2CH_2$ | O | Cl | O | H |
| 605 | t-Bu | Cl | O | $CH_2CH_2$ | O | Cl | O | 4-Me |
| 606 | t-Bu | Cl | O | $CH_2CH_2$ | O | Me | O | H |
| 607 | t-Bu | Cl | O | $CH_2CH_2$ | O | Me | O | 4-Cl |
| 608 | t-Bu | Cl | O | $CH_2CH_2$ | O | Me | O | 2-Me |
| 609 | t-Bu | Cl | O | $CH_2CH_2$ | O | Me | O | 4-Me |
| 610 | t-Bu | Cl | O | $CH_2CH_2$ | O | Me | O | 4-Et |
| 611 | t-Bu | Cl | O | $CH_2CH_2$ | O | Me | O | 4-t-Bu |
| 612 | t-Bu | Cl | O | $CH_2CH_2$ | O | Me | O | 4-OMe |
| 614 | t-Bu | Cl | O | $CH_2CH_2$ | O | Me | O | 4-$NO_2$ |
| 617 | t-Bu | Cl | O | $CH_2CH_2$ | O | Me | O | 2-Cl,4-Cl |
| 618 | t-Bu | Cl | O | $CH_2CH_2$ | O | Me | O | 2-Me,4-Me |

| No. | R | A | X | B | Y | $Z^1$ | Q | $Z^2n$ |
|---|---|---|---|---|---|---|---|---|
| 639 | t-Bu | Cl | O | $CH_2CH_2$ | O | H | CO | H |
| 640 | t-Bu | Cl | O | $CH_2CH_2$ | O | H | CO | 4-Cl |
| 642 | t-Bu | Cl | O | $CH_2CH_2$ | O | Cl | CO | H |
| 643 | t-Bu | Cl | O | $CH_2CH_2$ | O | Cl | CO | 4-Cl |
| 644 | t-Bu | Cl | O | $CH_2CH_2$ | O | Cl | CO | 4-Me |
| 645 | t-Bu | Cl | O | $CH_2CH_2$ | O | Me | CO | H |
| 646 | t-Bu | Cl | O | $CH_2CH_2$ | O | Me | CO | 2-Cl |
| 647 | t-Bu | Cl | O | $CH_2CH_2$ | O | Me | CO | 3-Cl |
| 648 | t-Bu | Cl | O | $CH_2CH_2$ | O | Me | CO | 4-Cl |
| 649 | t-Bu | Cl | O | $CH_2CH_2$ | O | Me | CO | 2-F |
| 650 | t-Bu | Cl | O | $CH_2CH_2$ | O | Me | CO | 4-F |
| 651 | t-Bu | Cl | O | $CH_2CH_2$ | O | Me | CO | 2-Br |
| 652 | t-Bu | Cl | O | $CH_2CH_2$ | O | Me | CO | 4-Br |
| 653 | t-Bu | Cl | O | $CH_2CH_2$ | O | Me | CO | 4-I |
| 654 | t-Bu | Cl | O | $CH_2CH_2$ | O | Me | CO | 2-Me |
| 655 | t-Bu | Cl | O | $CH_2CH_2$ | O | Me | CO | 3-Me |
| 656 | t-Bu | Cl | O | $CH_2CH_2$ | O | Me | CO | 4-Me |
| 657 | t-Bu | Cl | O | $CH_2CH_2$ | O | Me | CO | 4-Et |
| 658 | t-Bu | Cl | O | $CH_2CH_2$ | O | Me | CO | 4-Bu |
| 659 | t-Bu | Cl | O | $CH_2CH_2$ | O | Me | CO | 4-i-Pr |
| 660 | t-Bu | Cl | O | $CH_2CH_2$ | O | Me | CO | 4-t-Bu |

| No. | R | A | X | B | Y | $Z^1$ | Q | $Z^2n$ |
|---|---|---|---|---|---|---|---|---|
| 662 | t-Bu | Cl | O | $CH_2CH_2$ | O | Me | CO | 4-$NO_2$ |
| 664 | t-Bu | Cl | O | $CH_2CH_2$ | O | Me | CO | 4-OMe |
| 665 | t-Bu | Cl | O | $CH_2CH_2$ | O | Me | CO | 4-OEt |
| 668 | t-Bu | Cl | O | $CH_2CH_2$ | O | Me | CO | 2-F,4-F |
| 669 | t-Bu | Cl | O | $CH_2CH_2$ | O | Me | CO | 2-F,6-F |
| 670 | t-Bu | Cl | O | $CH_2CH_2$ | O | Me | CO | 2-Cl,4-Cl |
| 671 | t-Bu | Cl | O | $CH_2CH_2$ | O | Me | CO | 2-Cl,6-Cl |
| 672 | t-Bu | Cl | O | $CH_2CH_2$ | O | Me | CO | 3-Cl,4-Cl |
| 673 | t-Bu | Cl | O | $CH_2CH_2$ | O | Me | CO | 3-Cl,5-Cl |
| 674 | t-Bu | Cl | O | $CH_2CH_2$ | O | Me | CO | 2-Cl,4-$NO_2$ |
| 675 | t-Bu | Cl | O | $CH_2CH_2$ | O | Me | CO | 2-$NO_2$,4-Cl |
| 676 | t-Bu | Cl | O | $CH_2CH_2$ | O | Me | CO | 2-Me,4-Me |
| 677 | t-Bu | Cl | O | $CH_2CH_2$ | O | Me | CO | 2-Me,6-Me |
| 678 | t-Bu | Cl | O | $CH_2CH_2$ | O | Me | CO | 3-Me,4-Me |
| 679 | t-Bu | Cl | O | $CH_2CH_2$ | O | Me | CO | 3-Me,5-Me |
| 681 | t-Bu | Cl | O | $CH_2CH_2$ | O | Me | CO | 2-Cl,4-Me |
| 682 | t-Bu | Cl | O | $CH_2CH_2$ | O | Me | CO | 2-Cl,6-Me |
| 683 | t-Bu | Cl | O | $CH_2CH_2$ | O | Me | CO | 2-Me,4-Cl |
| 690 | t-Bu | Cl | O | $CH_2CH_2$ | O | i-Pr | CO | H |

| No. | R | A | X | B | Y | Z$^1$ | Q | Z$^2$n |
|---|---|---|---|---|---|---|---|---|
| 691 | t-Bu | Cl | O | $CH_2CH_2$ | O | H | $CH_2$ | H |
| 692 | t-Bu | Cl | O | $CH_2CH_2$ | O | H | $CH_2$ | 4-Cl |
| 693 | t-Bu | Cl | O | $CH_2CH_2$ | O | H | $CH_2$ | 4-Me |
| 694 | t-Bu | Cl | O | $CH_2CH_2$ | O | Cl | $CH_2$ | H |
| 695 | t-Bu | Cl | O | $CH_2CH_2$ | O | Cl | $CH_2$ | 4-Cl |
| 696 | t-Bu | Cl | O | $CH_2CH_2$ | O | Cl | $CH_2$ | 4-Me |
| 697 | t-Bu | Cl | O | $CH_2CH_2$ | O | Me | $CH_2$ | H |
| 698 | t-Bu | Cl | O | $CH_2CH_2$ | O | Me | $CH_2$ | 2-Cl |
| 699 | t-Bu | Cl | O | $CH_2CH_2$ | O | Me | $CH_2$ | 3-Cl |
| 700 | t-Bu | Cl | O | $CH_2CH_2$ | O | Me | $CH_2$ | 4-Cl |
| 701 | t-Bu | Cl | O | $CH_2CH_2$ | O | Me | $CH_2$ | 2-F |
| 702 | t-Bu | Cl | O | $CH_2CH_2$ | O | Me | $CH_2$ | 4-F |
| 703 | t-Bu | Cl | O | $CH_2CH_2$ | O | Me | $CH_2$ | 2-Br |
| 704 | t-Bu | Cl | O | $CH_2CH_2$ | O | Me | $CH_2$ | 4-Br |
| 705 | t-Bu | Cl | O | $CH_2CH_2$ | O | Me | $CH_2$ | 4-I |
| 706 | t-Bu | Cl | O | $CH_2CH_2$ | O | Me | $CH_2$ | 2-Me |
| 707 | t-Bu | Cl | O | $CH_2CH_2$ | O | Me | $CH_2$ | 3-Me |
| 708 | t-Bu | Cl | O | $CH_2CH_2$ | O | Me | $CH_2$ | 4-Me |
| 709 | t-Bu | Cl | O | $CH_2CH_2$ | O | Me | $CH_2$ | 4-Et |
| 710 | t-Bu | Cl | O | $CH_2CH_2$ | O | Me | $CH_2$ | 4-Bu |
| 711 | t-Bu | Cl | O | $CH_2CH_2$ | O | Me | $CH_2$ | 4-i-Pr |
| 712 | t-Bu | Cl | O | $CH_2CH_2$ | O | Me | $CH_2$ | 4-t-Bu |
| 714 | t-Bu | Cl | O | $CH_2CH_2$ | O | Me | $CH_2$ | 4-$NO_2$ |
| 716 | t-Bu | Cl | O | $CH_2CH_2$ | O | Me | $CH_2$ | 4-OMe |
| 717 | t-Bu | Cl | O | $CH_2CH_2$ | O | Me | $CH_2$ | 4-OEt |
| 720 | t-Bu | Cl | O | $CH_2CH_2$ | O | Me | $CH_2$ | 2-F,4-F |

| No. | R | A | X | B | Y | $Z^1$ | Q | $Z^2n$ |
|---|---|---|---|---|---|---|---|---|
| 721 | t-Bu | Cl | O | $CH_2CH_2$ | O | Me | $CH_2$ | 2-F,6-F |
| 722 | t-Bu | Cl | O | $CH_2CH_2$ | O | Me | $CH_2$ | 2-Cl,4-Cl |
| 723 | t-Bu | Cl | O | $CH_2CH_2$ | O | Me | $CH_2$ | 2-Cl,6-Cl |
| 724 | t-Bu | Cl | O | $CH_2CH_2$ | O | Me | $CH_2$ | 3-Cl,4-Cl |
| 725 | t-Bu | Cl | O | $CH_2CH_2$ | O | Me | $CH_2$ | 3-Cl,5-Cl |
| 726 | t-Bu | Cl | O | $CH_2CH_2$ | O | Me | $CH_2$ | 2-Cl,4-$NO_2$ |
| 727 | t-Bu | Cl | O | $CH_2CH_2$ | O | Me | $CH_2$ | 2-$NO_2$,4-Cl |
| 728 | t-Bu | Cl | O | $CH_2CH_2$ | O | Me | $CH_2$ | 2-Me,4-Me |
| 729 | t-Bu | Cl | O | $CH_2CH_2$ | O | Me | $CH_2$ | 2-Me,6-Me |
| 730 | t-Bu | Cl | O | $CH_2CH_2$ | O | Me | $CH_2$ | 3-Me,4-Me |
| 731 | t-Bu | Cl | O | $CH_2CH_2$ | O | Me | $CH_2$ | 3-Me,5-Me |
| 733 | t-Bu | Cl | O | $CH_2CH_2$ | O | Me | $CH_2$ | 2-Cl,4-Me |
| 734 | t-Bu | Cl | O | $CH_2CH_2$ | O | Me | $CH_2$ | 2-Cl,6-Me |
| 735 | t-Bu | Cl | O | $CH_2CH_2$ | O | Me | $CH_2$ | 2-Me,4-Cl |
| 742 | t-Bu | Cl | O | $CH_2CH_2$ | O | i-Pr | $CH_2$ | H |

The compound numbers in Tables 1 to 2 are referred to in preparation examples, formulation examples and test examples, which will be mentioned below.

The compounds of the present invention can be prepared by reacting a compound of the general formula (11):

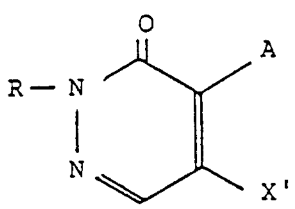

(II)

with a compound of the general formula (III):

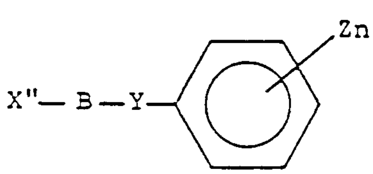

(III)

wherein R, A, B, Y, Z and n have the same meanings as defined above, and X' and X'' represent halogen atom, -SM or -OM in which M means hydrogen atom or alkali metal atom.

In general, it is preferable to use -SM or -OM as X'' in the general formula (III) when X' in the general formula (II) is halogen atom, and to use halogen atom as X'' in the general formula (III) when X' in the general formula (II) is -SM or -OM (wherein M represents hydrogen atom or alkali metal atom), with proviso that when X' is halogen atom, X'' represents -SM or -OM, when X' is -SM or -OM, X'' represents halogen atom.

It is also preferable to prepare the compounds of the present invention in the presence of appropriate bases and in solvents which do not affect the reaction. When M is alkali metal atom, the presence of the base is not necessarily needed.

As the solvents in the present invention can be used lower alcohols such as methanol, ethanol, etc.; ketones such as acetone, methylethylketone, etc.; hydrocarbons such as benzene, toluene, etc.; ethers such as isopropylether, tetrahydrofuran, 1,4-dioxane, etc.; amides such as N,N-dimethylformamide, hexamethyl phosphoric triamides, etc., halogenated hydrocarbons such as dichloromethane, dichloroethane, etc. As necessary, mixtures of these solvens or mixtures these solvents and water can also be used.

As the base can be used inorganic bases such as sodium hydride, sodium hydroxide, potassium hydroxide, sodium carbonate, potassium carbonate, sodium hydrogen-carbonate, etc., and organic bases such as sodium methoxide, sodium ethoxide, triethylamine, pyridine, etc. If necessary, tetraammonium salts, for example, triethylbenzylammonium chloride, etc. may be added to the reaction system as catalyst.

The reaction temperature may be in a range of from -20 °C to a boiling point of the solvent to be used in the reaction. The reaction temperature is preferable in a range of from - 5 °C to a boiling point of the solvent to be used in the reaction.

The ratio of the raw material can he optionally selected, but it is advantageous to conduct the reaction using equimolar or nearly equimolar amount of the materials.

Preparation of the compound of the present invention is described more in detail by the way of the following examples which are not to restrict to the invention.

Preparation Example 5

Preparation of 2-t-butyl-4-chloro-5- (2'-(2'',6''-dimethylphenoxy) ethylthio) -3-(2H)-pyridazinone (Compound No. 24)

In 30 mm of N,N-dimethylformamide was dissolved 2.2 g of 2-t-butyl-4-chloro-5-mercapto-3(2H)-pyridazinone and 2.3 g of 2-(2',6'-dimethylphenoxy)ethyl bromide, and thereto were added 1.1 g of anhydrous sodium carbonate. The resulting solution was stirred at room temperature for fifteen hours. This solution was poured into water and extracted with diethyl ether. The organic layer was dried over anhydrous sodium sulfate and freed of solvent under reduced pressure to give crystals. The crystals thus obtained were washed with n-hexane to give 3.3 g of the intended compound. m.p. 104.3 ~ 105.2 °C

Preparation Example 6

Preparation of 2-t-butyl-4-chloro-5- (2'-(2'',6''-dimethyl-4''-butylcarbonylphenoxy)ethylthio ) -3(2H)-pyridazinone (Compound No. 78)

In 30 mm of N,N-dimethylformamide was dissolved 2.2 g of 2-t-butyl-4-chloro-5-mercapto-3(2H)-pyridazinone and 3.0 g of 2-(2',6'-dimethyl-4'-butylcarbonylphenoxyethyl bromide and thereto were added 1.5 g of anhydrous sodium carbonate. The resulting solution was stirred at room temperature for fifteen hours. This solution was poured into water and extracted with diethyl ether. The organic layer was dried over anhydrous sodium sulfate and freed of solvent under reduced pressure to give a crude product. The crude product thus obtained was purified by means of column chromatography (on silica gel, eluting with benzene) to give 3.3 g of the intended compound as an oil.

Preparation Example 8

Preparation of 2-t-butyl-4-chloro-5- ( 2'-(2''-methyl-4''-pentyl-phenoxy)ethoxy ) -3-(2H)-pyridazinone (Compound No. 220)

In 30 ml of N,N-dimethylformamide were dissolved 2 g of 2-t-butyl-4-chloro-5-hydroxy-3(2H)-pyridazinone and 2.8 g of 2-(2'-methyl-4'-pentylphenoxy)ethyl bromide, and thereto was added 2 g of anhydrous potassium carbonate. The reaction mixture was stirred at 80 °C for three hours. The resulting solution was poured into water and extracted with diethylether. The organic layer was dried over anhydrous sodium sulfate and freed of solvent by distillation under reduced pressure to obtain a crude product. The crude product thus obtained was purified by means of column chromatography (on silica gel, eluting with benzene) to give 3.3 g of the intended compound as an oil.

Preparation Example 11

Preparation of 2-t-butyl-4-chloro-5- ( 2'-(2'',6''-dimethyl-4''-benzoylphenoxy)ethylthio ) -3-(2H)-pyridazinone (Compound No.465)

In 50 ml of N,N-dimethylformamide were dissolved 6.6 g of 2-t-butyl-4-chloro-5-mercapto-3(2H)-pyridazinone and 10 g of 2-(2',6'-dimethyl-4'-benzoylphenoxy)ethyl bromide, and thereto was added 5 g of anhydrous sodium carbonate. The solution was stirred at room temperature for seventeen hours. The resulting solution was poured into water and extracted with diethyl ether. The organic layer was dried over anhydrous sodium sulfate and freed of solvent by distillation under reduced pressure to give a crude product. The crude product thus obtained was purified by means of column chromatograpy (on silica gel, eluting with benzene) and crystallization (from 50 ml of n-hexane-diethyl ether (4 : 1)) to give 13.3 g of the intended compound.
m.p. 94.6 ~ 96.2 ° C

The physical properties of the compounds prepared according to one of the methods of the Preparation Examples 5,6,8 and 11 are shown in the following Table 4.

Table 4

| No. | m.p. (℃) |
|---|---|
| 12 | 109.2 — 111.6 |
| 13 | oil |
| 14 | oil |
| 15 | oil |
| 16 | 61.0 — 63.0 |
| 17 | 65.0 — 70.0 |
| 18 | oil |
| 19 | oil |
| 21 | 87.0 — 88.5 |
| 22 | 96.8 — 98.5 |
| 23 | 112.4 — 113.3 |
| 24 | 104.3 — 105.2 |
| 25 | oil |
| 26 | 78.3 — 80.7 |
| 27 | 98.6 — 102.1 |
| 28 | oil |
| 29 | oil |
| 30 | oil |
| 31 | 95.7 — 98.0 |
| 32 | 109.0 — 111.2 |
| 33 | 106.6 — 109.2 |
| 34 | 95.4 — 97.0 |
| 36 | oil |
| 38 | 123.4 — 127.3 |
| 40 | 94.0 — 96.0 |
| 41 | oil |
| 44 | oil |
| 45 | oil |
| 46 | 115.0 — 118.1 |
| 49 | oil |
| 53 | oil |
| 55 | 92.8 — 93.6 |
| 70 | oil |
| 71 | 152.9 — 153.7 |
| 72 | 116.9 — 118.2 |
| 73 | 106.9 — 108.6 |
| 74 | 127.9 — 130.1 |
| 76 | oil |
| 78 | oil |
| 79 | oil |
| 80 | oil |
| 82 | 107.0 — 108.8 |
| 84 | 79.7 — 80.8 |
| 85 | 90.0 — 91.5 |

| No. | m.p. (℃) |
|---|---|
| 88 | 103.5 — 104.8 |
| 89 | 119.8 — 124.5 |
| 92 | 117.2 — 120.1 |
| 93 | oil |
| 94 | 108.2 — 109.2 |
| 95 | oil |
| 97 | oil |
| 98 | oil |
| 112 | 107.7 — 108.3 |
| 113 | 86.2 — 88.5 |
| 148 | oil |
| 150 | oil |

| No. | m.p. (℃) |
|---|---|
| 202 | 85.0 — 87.0 |
| 203 | 90.9 — 92.6 |
| 204 | 86.0 — 88.1 |
| 205 | oil |
| 206 | 69.7 — 72.5 |
| 207 | oil |
| 208 | 80.3 — 81.6 |
| 210 | 122.5 — 124.3 |
| 212 | oil |
| 213 | 78.5 — 80.5 |
| 216 | 122.1 — 125.1 |
| 217 | 117.0 — 119.0 |
| 218 | 105.0 — 109.0 |
| 219 | 126.5 — 127.7 |
| 220 | oil |
| 221 | oil |
| 222 | 117.9 — 119.4 |
| 223 | 111.1 — 113.5 |
| 224 | 97.0 — 101.0 |

| No. | m.p. (℃) | No. | m.p. (℃) |
|---|---|---|---|
| 225 | oil | 283 | 86.7 — 88.1 |
| 226 | 124.6 — 129.5 | 284 | 108.8 — 110.5 |
| 228 | 131.1 — 132.6 | 287 | 173.3 — 176.3 |
| 229 | 133.2 — 134.7 | 288 | 86.6 — 87.9 |
| 230 | 120.0 — 124.0 | 289 | 147.1 — 148.3 |
| 232 | 79.0 — 80.3 | 290 | 73.1 — 75.5 |
| 233 | 142.5 — 146.5 | 292 | oil |
| 235 | 85.0 — 87.0 | 293 | 64.1 — 64.2 |
| 236 | 96.0 — 98.0 | | |
| 239 | 155.7 — 157.4 | 343 | 96.1 — 97.3 |
| 240 | oil | 344 | 117.9 — 120.9 |
| 241 | 108.2 — 111.8 | | |
| 248 | 149.5 — 151.2 | | |
| 250 | 137.9 — 139.2 | | |
| 265 | 112.9 — 114.5 | | |
| 266 | 171.0 — 173.8 | | |
| 267 | 150.6 — 152.9 | | |
| 268 | 131.5 — 133.5 | | |
| 269 | 135.2 — 137.8 | | |
| 271 | 116.1 — 117.6 | | |
| 273 | 122.0 — 124.8 | | |
| 275 | 98.1 — 104.2 | | |
| 277 | 143.1 — 145.6 | | |
| 279 | 107.6 — 109.5 | | |
| 280 | 102.8 — 106.5 | | |

| No. | m.p. (℃) |
|---|---|

| No. | m.p. (℃) |
|---|---|
| 421 | 120.3 — 128.2 |
| 463 | 150.2 — 151.8 |
| 465 | 94.6 — 96.2 |
| 466 | 156.9 — 158.1 |
| 467 | oil |
| 468 | 142.2 — 144.1 |
| 470 | oil |
| 472 | 153.1 — 154.5 |
| 474 | 129.2 — 131.4 |
| 475 | oil |
| 476 | oil |
| 480 | 138.0 — 140.0 |
| 482 | 162.0 — 164.6 |
| 484 | 125.1 — 126.4 |
| 490 | 108.9 — 111.2 |

| No. | m.p. (℃) | No. | m.p. (℃) |
|---|---|---|---|
| 492 | 126.5 — 128.0 | 672 | 143.0 — 144.8 |
| 493 | 158.7 — 161.3 | 673 | 177.5 — 179.0 |
| 494 | 134.4 — 135.4 | 676 | 122.7 — 123.4 |
| 496 | 121.7 — 122.5 | 678 | 157.6 — 159.1 |
| 498 | 113.8 — 115.9 | | |
| | | 694 | 139.2 — 140.4 |
| 514 | oil | 697 | 163.4 — 165.0 |
| 517 | 123.1 — 126.1 | 698 | 179.8 — 182.2 |
| 518 | 136.4 — 137.5 | 699 | 116.7 — 117.7 |
| 519 | 98.3 — 99.2 | 722 | 58.0 — 62.0 |
| 520 | oil | 725 | 130.0 — 131.0 |
| 527 | 105.9 — 107.6 | | |
| 542 | 115.2 — 116.4 | | |
| 545 | 130.7 — 132.5 | 111 | 91.1 — 92.1 |
| | | 491 | 212.6 — 213.6 |
| | | 477 | 116.6 — 118.6 |
| | | 306 | 125.9 — 127.6 |
| 640 | 109.9 — 112.3 | 671 | 224.5 — 226.5 |
| | | 657 | 137.6 — 139.1 |
| 643 | 156.8 — 158.0 | | |
| 646 | 202.3 — 204.0 | 489 | 161.0 — 163.6 |
| 647 | 135.8 — 137.8 | 83 | 121.0 — 122.0 |
| 652 | 163.9 — 166.0 | 278 | 142.7 — 144.7 |
| 654 | 168.9 — 170.1 | | |
| 655 | 134.1 — 135.9 | | |
| 656 | 113.0 — 116.0 | | |
| 660 | 143.9 — 145.6 | | |
| 662 | 193.0 — 196.0 | | |
| 664 | 168.6 — 170.9 | | |
| 670 | 153.0 — 154.3 | | |

When the compounds according to the present invention can be used for insecticidal, acaricidal, nematicidal and/or fungicidal agents for agricultural and horticultural uses or for expellents of ticks parasitic on animals, they are generally mixed with appropriate carriers, for instance, solid carriers such as clay, talc, bentonite or diatomaceous earth, or liquid carriers such as water, alcohols (e.g., methanol and ethanol, aromatic hydrocarbons (e.g., benzene, toluene and xylene), chlorinated hydrocarbons, ethers, ketones, acid amides (e.g., dimethylformamide (DMF)) or esters (e.g., ethyl acetate). If desired, to these mixtures can be added a surfactant, emulsifier, dispersing agent, suspending agent, penetrating agent, spreader, stabilizer and the like to put them into practical uses in the form of liquid preparation, emulsifiable concentration,

wettable powder, dust, granule, flowable or the like. Moreover, the resulting mixtures may be incorporated with other herbicides, various insecticides, fungicides, plant-growth regulating agents and/or synergists during preparation or application thereof, as necessary.

The amount of the compounds of the invention to be used as an active ingredients is suitably in the range of 0.005 to 50 kg per hectare although it varies depending upon the place and the season where the compounds are applied, manner of application, diseases and insect pests to be applied, cultivated crops to be protected and the like.

In the following, there are shown component ratios of formulations and formulation examples of fungicidal, insecticidal, acaricidal and/or nematicidal compositions and expellent compositions for ticks parasitic on animals, said compositions containing the compounds of the present invention as an active ingredient. These examples are merely illustrative and not to restrict the invention. In the following examples, "part" means "part by weight" and "%" means "% by weight".

1. Components Ratio of Formulation

| (1) Emulsifiable concentrates | |
|---|---|
| Active ingredient: | 5 - 25 wt % |
| liquid carrier: | 52 - 90 wt % (Xylene, DMF, Methyl naphthalene, Cyclohexanone, Dichlorobenzene, Isophorone) |
| surface active agent: | 5 - 20 wt % (Sorpol 2680, Sorpol 3005X, Sorpol 3346) |
| others: | 0 - 20 wt % (Piperonyl butoxide: 0 - 20 wt %) Benzotriazole: 0 - 5 wt %) |

| (2) Oil solutions | |
|---|---|
| Active ingredient:<br>liquid carrier: | 5 - 30 wt %<br>70 - 95 wt % (Xylene, Methyl cellosolve, Kerosene) |

(3) Flowables

| | |
|---|---|
| Active ingredient: | 5 - 70 wt % |
| liquid carrier: | 12.4 - 78.4 wt % (water) |
| surface active agent: | 1 - 10.5 wt % (Lunox 1000C, Sorpol 3353, Soprophor FL, Nippol, Agrisol S-710, Lignin sulfonic acid soda) |
| others: | 0 - 10 wt % (Formalin 0-0.3 wt %, Ethylene glycol 0 - 10 wt %, Propylene glycol 0 - 10 wt %) |

| (4) Wettable powders (W.P.) | |
|---|---|
| Active ingredient: | 5 - 70 wt % |
| Solid carrier: | 15 - 89 wt % (Calcium carbonate, Kaolinite, Zeeklite D, Zeeklite PFP, Diatomaceous earth, Talc) |
| surface active agent: | 3 - 10 wt % (Sorpol 5039, Lunox 1000C, Sulfonic acid calcium, Sodium dodecyl-sulfonate, Sorpol 5050, Sorpol 005D, Sorpol 5029-0) |

| (5) Dusts | |
|---|---|
| Active ingredient: | 0.01 - 30 wt % |
| solid carrier: | 67 - 99 wt % (Calcium carbonate, Kaolinite, Zeeklite, Talc) |
| others: | 0 - 3 wt % (Diisopropylphosphate: 0-1.5 wt %, Carplex #80: 0-3 wt %) |

| (6) Granules | |
| --- | --- |
| Active ingreident:<br>solid carrier:<br>others: | 0.1 - 30 wt %<br>67 - 99 wt % (Calcium carbonate, Kaolinite, Talc, Bentonite)<br>0 - 8 wt % (Calcium lignin sulfonate: 0-3 wt %, Polyvinylalcohol: 0-5 wt %) |

2. Formulation Examples

| Formulation Example 1: Emulsifiable concentrates | |
|---|---|
| Active ingredient | 20 parts |
| Xylene | 55 parts |
| N,N-dimethylformamide | 20 parts |
| Solpol 2680 (trade name, a mixture of a non-ionic surface-active agent and an anionic surface-active agent manufactured by Toho Chemicals, Co., Ltd., Japan) | 5 parts |

The above components are mixed intimately together to form an emulsifiable concentrate. Upon use, the emulsifiable concentrate is diluted with water up to one fiftieth to one twenty thousandth in concentration and applied at a rate of 0.005 to 50 kg of the active ingredient per hectare.

| Formulation Example 2: Wettable powders | |
|---|---|
| Active ingredient | 25 parts |
| Zeeklite PFP (trade name, a mixture of kaolinite and sericite manufactured by Zeeklite Mining Industries Co., Ltd.) | 66 parts |
| Solpol 5039 (trade name, an anioic surface-active agent manufactured by Toho Chemical Co., Ltd., Japan) | 4 parts |
| Carplex # 80 (trade name, white carbon manufactured by Shionogi Seiyaku K.K., Japan) | 3 parts |
| Calcium lingin sulfonate | 2 parts |

The above components are homogeneously mixed together and ground to form a wettable powder. Upon use, the wettable powder is diluted with water up to one fiftieth to one twenty thousandth and applied at a rate of 0.005 to 50 kg of the active ingredient per hectare.

| Formulation Example 3: Oil solutions | |
|---|---|
| Active ingredient | 10 parts |
| methylcellosolve | 90 parts |

The above components are homogeneously mixed together to form an oil solution. Upon use, the oil solution is applied at a rate of 0.005 to 50 kg of the active ingredient per hectare.

| Formulation Example 4: Dusts | |
|---|---|
| Active ingredient | 3.0 parts |
| Carplex #80 (trade name, white carbon manufactured by Shionogi Seiyaku K.K., Japan) | 0.5 parts |
| Clay | 95.0 parts |
| di-isopropyl phosphate | 1.5 parts |

The above components are homogeneously mixed together and ground to form a dust. Upon use, the dust is applied at a rate of 0.005 to 50 kg of the active ingredient per hectare.

| Formulation Example 5: Granules | |
|---|---|
| Active ingredient | 5 parts |
| Bentonite | 54 parts |
| Talc | 40 parts |
| Calcium lignin sulfonate | 1 part |

The above components are mixed intimately together and ground, incorporated with a small amount of water and mixed together with stirring. The resulting mixture is granulated by means of extrusion-granulator and dried to form granules. Upon use, the granule is applied at a rate of 0.005 to 50 kg of the active ingredient per hectare.

Formulation Example 6: Flowables

| | |
|---|---|
| Active ingredient | 25 parts |
| Solpol 3353 (trade name, a nonionic surface-active agent manufactured by Toho Chemical Co., Ltd., Japan) | 10 parts |
| Runox 1000C (trade name, an anionic surface-active agent manufactured by Toho Chemical Co., Ltd., Japan) | 0.5 part |
| 1% aqueous solution of Xanthan gum (natural high-molecular compound) | 20 parts |
| Water | 44.5 parts |

The above components except the active ingredient are homogeneously mixed together to form a solution, and thereto is added the active ingredient. The resulting mixture is throughly stirred, wet-ground by means of sand mill to form a flowable. Upon use, the flowable is diluted up to one fiftieth to one twenty thousandth with water and applied at a rate of 0.005 to 50 kg of the active ingredient per hectare.

The compounds according to the present invention not only exhibit superior insecticidal action on hemiptera insect such as green rice leafhopper (Nephotettix cincticeps), lepidoptera insect such as diamondback moth (Plutella xylostella), Coleoptera and sanitary insect pests such as pale house mosquito (Culex pipiens ), but are also useful for expelling mites parasitic on fruits and vegetables such as two-spotted spinder mite ( Tetranychus urticae ), Kanzawa spinder mite (Tetranychus kanzawai), Carmine mite (Tetranychus cinnabarinus), citrus red mite (Panonychus citri ) and European red mite (Panonychus ulmi), as well as ticks parasitic on animals such as southern cattle tick (Boophilus microplus ), cattle tick ( Boophilus annulatus ), galf coast tick (Amblyomma maculatum ), brown-ear tick (Rhipicephalus appendiculatus) and Haemaphysalis longicornis ). The main features of the compounds of the present invention resides in that the compounds are useful for the prevention or control of blight (or disease) of fruits and vegetables such as powdery mildew, downy mildew, rust, rice blast disease (or rice blight) etc. in addition to having the above mentioned insecticidal, acaricidal and nematicidal actions. Accordingly, the compounds of the present invention are an excellent agricultural drug which enables control of pests and blight (or disease) simultaneously. Moreover, they are excellent as an expellent for ticks parasitic on animals such as domestic animals (e.g. cattle, horse, sheep and pig), domestic fowls, and other animals such as dog, cat, rabbit and the like.

The present invention is further explained in detail by way of the following test example.

Test Example 1: Insecticidal test on Green rice leafhopper (Nephotettix cincticeps)

20% emulsifiable concentrate of each of the present compounds described in the specification (some of which were applied with 25% wettable powder) was diluted with water containing an extender to prepare a 1000 ppm aqueous emulsion. This aqueous emulsion was dispersed in full amount to stems and leaves of paddy planted in a pot of 1/20000 are and air-dried. Twenty second instar green rice leafhopper larvae which would show resistance to organic phosphorus type insecticides or carbamate type insecticides, per pot, were released in the pots. The paddies were covered with a cylindrical wire gauge and then placed in a thermostatic chamber. The numbers of the larvae killed was determined after 96 hours and the mortality

thereof was calculated according to the equation below. Incidentally, the test was repeated twice for each compound.

$$\text{Mortality (\%)} = \frac{\text{number of the insect killed}}{\text{number of the insect released}} \times 100$$

As the results, the following compounds exhibited 100 % of mortality.

Compound Nos. 14, 16, 17, 21, 22, 24, 25, 26, 29, 30, 31, 32, 33, 36, 38, 40, 41, 44, 45, 49, 53, 71, 72, 73, 76, 78, 79, 80, 82, 83, 84, 85, 88, 89, 92, 93, 97, 98, 111, 112, 113, 148 150, 202, 203, 204, 206, 207, 208, 213, 217, 220, 221, 226, 232, 235, 236, 239, 240, 267, 268, 269, 273, 277, 279, 280, 283, 284, 288, 292, 465, 466 467, 468, 470, 472, 474, 475, 476, 477, 482, 484, 489, 490, 492, 494, 496, 498, 514, 517, 518, 519, 520, 527, 529, 542, 640, 643, 647, 652, 656, 657, 664, 670, 672, 676, 722

Test Example 2: Insecticidal test on 28-spotted Lady beetle (Henosepilachna vigintioctopunctata)

A leaf of tomato was immersed for about 10 seconds in a 1000 ppm aqueous emulsion which had been prepared by diluting with water containing an extender 20 % emulsifiable concentrate containing each compound described in the present specification (some of which were applied with 25 % wettable powder) and then air-dried. The leaf thus treated was placed in a laboratory dish, into which 10 second inster 28-spotted lady beetle larvae were released. The dish was then fitted with a cap provided with pores and then placed in a thermostatic chamber kept at 25 °C. The number of the larvae killed was determined after 96 hours and the mortality thereof was calculated according to the equation described in the Test Example 1. Incidentally, the test was repeated twice for each compound.

As the results, the following compounds exhibited 100 % of mortality.

Compound Nos. 13, 14, 16, 17, 18, 21, 24, 25, 26, 29, 30, 31, 32, 33, 34, 36, 38, 40, 41, 44, 45, 46, 49, 53, 55, 70, 71, 72, 73, 74, 76, 78, 79, 80, 82, 83, 84, 85, 88, 89, 92, 93, 94, 95, 97, 99, 111, 112, 113, 150, 202, 203, 204, 206, 208, 212, 216, 217, 218, 220, 221, 226, 232, 235, 236, 248, 250, 268, 269, 279, 280, 283, 284, 288, 290, 463, 465, 466, 467, 468, 470, 472, 474, 475, 476, 477, 480, 482, 484, 489, 490, 492, 494, 496, 498, 514, 517, 518, 519, 520 527, 529, 542, 643, 652, 657, 662, 664, 722

Test Example 3: Acaricidal test on Kanzawa Spider Mite (T. Kanzawai )

A leaf of kidney bean was cut into a round piece of 1.5 cm in diameter by a leaf punch, and then placed on a moistened filter paper on a polystyrol cup of 7 cm in a diameter. Each piece of the leaf was inoculated with 10 Kanzawa Spinder Mite nymphs. Half a day after the inoculation, each 2 ml of an aqueous emulsion containing 1000 ppm of the present compound described in the specification (some of which were applied with 25 % wettable powder) diluted with water containing an extender was applied to each polystyrol cup by means of a rotary spray tower. After 96 hours, the mortality of the nymph was determined according to the equation described in the Text Example 1. Incidentally, the test was repeated twice for each compound.

As the results, the following compounds exhibited 100 % of mortality.

Compound Nos. 13, 14, 15, 16, 17, 18, 24, 25, 26, 28, 29, 30, 31, 32, 33, 36, 40, 41, 44, 45, 49, 53, 55, 70, 74, 76, 78, 80, 83, 84, 85, 88, 89, 92, 93, 95, 97, 98, 111, 112, 113, 148, 150, 203, 204, 208, 212, 220, 221, 224, 226, 232, 235, 236, 240, 241, 250, 283, 287, 288, 290, 344, 421, 463, 466, 467, 468, 470, 472, 475, 476, 477, 480, 482, 489, 490, 494, 514, 517, 518, 519, 520, 527, 529, 542, 601, 643

Test Example 4: Nematicidal test on Root-knot Nematode (Meloidogyne sp.)

Soil contaminated with root-knot nematode was placed in a polystyrol cup 8 cm in diameter. A liquid containing 1000 ppm of an active ingredient was prepared by diluting 20 % emulsifiable concentrate containing a compound of the present invention (some of which were applied with 25 % wettable powder) with water containing an extender. The soil contaminated with nematode and placed in the polystyrol cup was drenched with each 50 ml of the resulting liquid. After 48 hours, a tomato seedling as an indicator was transplanted into the soil thus treated. Thirty days after the transplantation, the roots of the tomato were

washed with water and the root-knot parasitism was checked by observation. Incidentally, the test was repeated twice for each compound. As a result, no root-knot parasitism was obserbed in the roots of the tomato treated with the exhibit a strong nematicidal activity:

Compound Nos. 17, 72, 73, 76, 78, 84, 85, 92, 93, 208, 221, 268, 269, 280, 283, 465

Test Example 5: Test for controlling Downy mildew of cucumber

Employing cucumbers (Cucumis sativus L.: variety sagamihanjiro) which had been grown for 2 weeks, thereto was sprayed a solution of an emulsifiable concentrate containing present compound which had been adjusted to a predetermined concentration (1000 ppm) at the rate of 20 ml per pot. After each pot was placed for one day in a green house, a suspension of spores of Pseudopernonospora cubensis (the concentration of the spores being such that 15 pieces of the spore can be obserbed by a 150 magnification microscope) was sprayed to the cucumbers for inoculation. The cucumbers to which the spores of Pseudoperonospora cubensis had been inoculated were left for 24 hours in a room kept at 25 °C relative humidity of 100 % and then transported to a green house for observation of disease appearance. Seven days after the inoculation, the percentage of the disease appearance were measured. As the results, no disease appearance was observed at all with respect to the following compounds.

Compound Nos. 22, 24, 28, 29, 30, 32, 33, 34, 36, 40, 45, 49, 55, 71, 72, 74, 76, 78, 85, 92, 95, 112, 113, 202, 205, 208, 219, 240, 279, 283, 288, 421, 463, 466, 467, 468, 475, 490, 514, 517, 518, 519, 640, 643, 670, 694, 699

Test Example 6: Test for controlling Powdery mildew of cucumber

Employing cucumbers (Cucumis sativus L.: variety sagamihanjiro) which had been grown in pots for 2 weeks, thereto was sprayed a solution of an emulsifiable containing a compound of the present invention and which had been adjusted to a predetermined concentration (1000 ppm) at the rate of 20 ml per pot. After each pot was placed for one day in a green house, a suspension of spores Sphaerotheca fulginea (the concentration of the spores being such that 25 pieces of the spores can be observed by a 150 magnification microscope) was sprayed to the cucumbers for inoculation. The cucumbers were placed in a green house at 25 - 30 °C for observation of disease appearance. Ten days after the inoculation, the percentages of the disease appearances were measured and evaluated. As the results, no disease appearance was observed at all with respect to the following compounds.

Compound Nos. 21, 22, 24, 25, 29, 72, 74, 76, 78, 79, 85, 89, 92, 93, 112, 113, 205, 207, 208, 213, 216, 217, 219, 220, 226, 280, 283, 284, 467, 470, 475, 517, 694, 697, 699

Test Example 7: Test for controlling Leaf rust of wheat

To wheats (Norin No. 61, third to fourth leaf-stage) which had been grown in pots of 9 cm in diameter was sprayed a solution of an emulsifiable concentrate containing a compound of the invention and which had been adjusted to a concentration of 1000 ppm at the rate of 20 ml per pot by means of a spray gun. The next day, thereto was sprayed a suspension of spores of Puccinia recondita (the concentration of the suspension being such that 30 spores can be observed by a 150 magnification) for inoculation. The wheats to which the spores of Puccinia recondita had been inoculated were left in a box kept at 25°C with a relative humidity of not lower than 95 % for one day and then allowed to stand at room temperature. Ten days after the inoculation, the disease area thus formed was measured and the protective value of each compound was calculated according to the following equation:

$$\text{Protective value (\%)} = \left(1 - \frac{\text{disease area in a treated plot)}}{\text{disease area in a non-treated plot}}\right) \times 100$$

As the results, the following compounds exhibited 100 % of protective value.

Compound Nos. 72, 74, 78, 79, 80, 82, 85, 89, 95, 97, 98, 113, 207, 292, 468

Test Example 8: Test for controlling Rice Blast

To rice plants (Nihonbare, third leaf-stage) which had been grown in pots was sprayed a solution of an emulsifiable concentrate which contains a compound of the present invention in 1000 ppm at the rate of 20 ml per pot by means of a spray gun. The next day, a suspension of spores of Pyricularia oryzae - (concentration of the suspension being such that 30 pieces of the spore can be observed by a 150 magnification microscope) was sprayed to the rice plants. Each pot was placed in an inoculation box kept at 25 °C with a relative humidity of not lower than 95 % for one day and thereafter allowed to stand at room temperature. Seven days after the inoculation, the percentage of the disease appearance were measured and evaluated. As the results, no disease appearance was observed at all with respect to the following compounds:

Compound Nos . 15, 19, 70, 89, 92, 112, 113, 205, 232, 269, 284, 288, 289, 475, 498

Test Example 9: Insectical test on Green rice leafhopper (Nephotettix cincticeps) (Comparative test)

Test was conducted in accordance with Test Example 1 except that the concentration each of the present compound and a control compound was changed to 500 ppm. The results are shown in Table 5. From the results, it can be seen that the present compounds exhibit much higher insecticidal activity than the known control compounds.

Test Example 10: Insectical test on 28-spotted Lady Beetle (Henosepilachna vigintioctopunctata) (Comparative test)

Test was conducted in, accordance with Test Example 2 except that the concentration each of the present compound and a control compound was changed to 500⁻ ppm. The results are shown in Table 5. From the results, it can be seen that the present compounds exhibit much higher insecticidal activity than the known control compounds.

Test Example 11: Acaricidal test on Kanzawa Spider Mite (T. Kanzawai ) (Comparative test)

Test was conducted in accordance with Test Example 3 except that the concentration each of the present compound and a control compound was changed to 500 ppm. The results are shown in Table 5. From the results, it can be seen that the present compounds exhibit much higher acaricidal activity than the known control compounds.

Table 5

| Insecticidal Test (Comparative test) | | | |
|---|---|---|---|
| Compound No. | Green rice leafhopper (mortality %) | 28-spotted lady beetle (mortality %) | Kanzawa spider mite (mortality %) |
| Present compound No. 40 | 100 | 100 | 100 |
| Present compound No. 92 | 100 | 100 | 100 |
| Present compound No. 465 | 100 | 100 | 100 |
| Control compound A | 0 | 20 | 0 |
| Control compound B | 20 | 0 | 50 |

The structural formulae of the present compounds in Table 5 are as follows:

No. 40

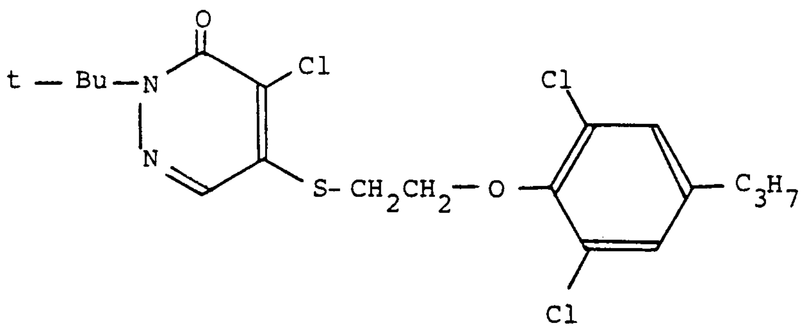

No. 92

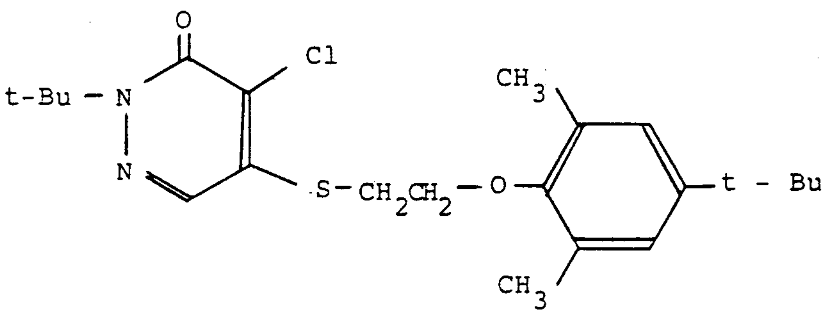

No. 465

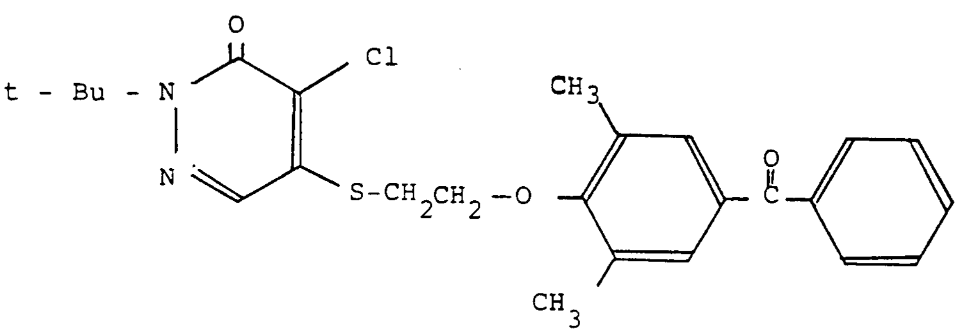

On the other hand, the structural formulae of the control compounds are as follows:

Compound A

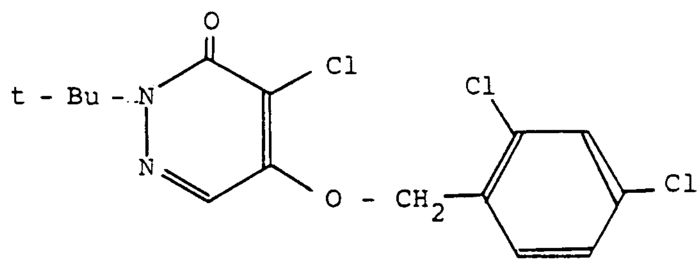

(compounds described in EP-A-0088384)

Compound B

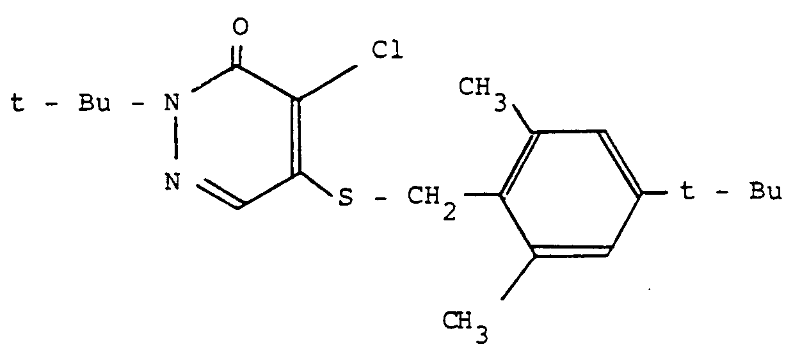

(compounds described in EP-A-0134439)

(Note that the above-mentioned "t-Bu" represents tartiary butyl group.)

Test Example 12: Residual activity text on 28-spotted lady beetle (Henosepilachna Vigintioctopunctata )

Tomato planted in a pot of 1/5000 are was sprayed well with a 500 ppm aqueous emulsion which had been prepared by diluting with water containing an extender 20 % emulsifiable concentrate containing each compound described in the present specification (some of which were applied with 25 % wettable powder), air-dried and kept in a greenhouse. After 10 days, leaf of the tomato thus treated was cut out and placed in a laboratory dish, into which 10 second inster 28-spotted lady beetle larvae were released. The dish was then fitted with a cap provided with pores and then placed in a thermostatic chamber kept at 25 ° C. The number of the larvae killed was checked after 96 hours and the mortality thereof was calculated according to the equation described in the Test Example 1. Incidentally, the test was repeated twice for each compound.

As the results, the following compounds exhibited 100 % of morality.

Compound Nos. 72, 84, 85, 89, 112, 113, 465, 468, 470, 472, 490, 496, 517, 520

## Claims

1. A pyridazinone derivative of the formula (I):

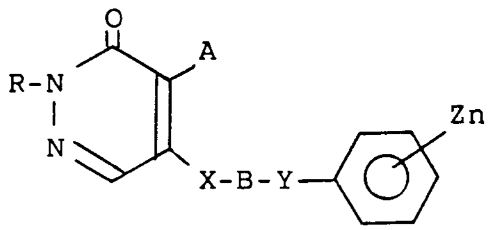

(I)

wherein

R represents tertiary butyl,

A represents chlorine atom,

B represents $-CH_2-CH_2-$,

X represents oxygen atom or sulfur atom,

Y represents oxygen atom,

n is an integer of 1 to 5 and when n is 2 to 5, Z may be same or different,

Z represents halogen atom, straight or branched chain alkyl group having 1 to 10 carbon atoms, straight or branched chain alkoxy group having 1 to 10 carbon atoms, alkylcarbonyl having 2 to 10 carbon atoms,

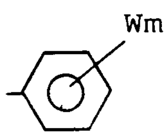 , 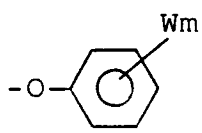 , 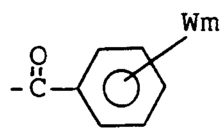 or 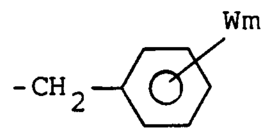

W represents halogen atom, alkyl group having 1 to 4 carbon atoms, alkoxy group having 1 to 4 carbon atoms or nitro group,

m is 0 or an integer of 1 to 2, and when m is 2, W may be same or different.

2. A process for preparing pyridazinone derivative of the general formula (I):

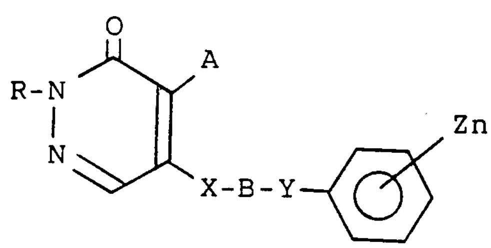

(I)

wherein

R represents tertiary butyl,

A represents chlorine atom,

B represents $-CH_2-CH_2-$,

X represents oxygen atom or sulfur atom,

Y represents oxygen atom,

n is an integer of 1 to 5 and when n is 2 to 5, Z may be same or different,

Z represents halogen atom, straight or branched chain alkyl group having 1 to 10 carbon atoms, straight or branched chain alkoxy group having 1 to 10 carbon atoms, alkylcarbonyl having 2 to 10 carbon atoms,

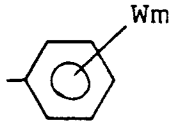 , 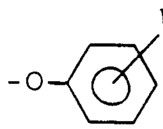 , 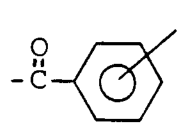 or 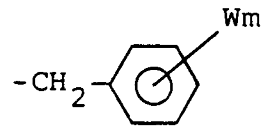

W represents halogen atom, alkyl group having 1 to 4 carbon atoms, alkoxy group having 1 to 4 carbon atoms or nitro group,

m is 0 or an integer of 1 to 2, and when m is 2, W may be same or different, which comprises reacting a compound of the formula (II):

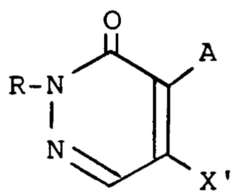

(II)

with a compound of the formula (III):

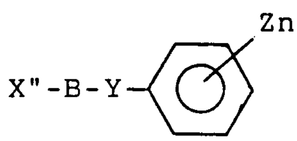

(III)

wherein R, A, B, Y, Z and n have the same meanings as defined above, and X' and X" represent halogen atom, -SM or -OM in which M means hydrogen atom or alkali metal atom.

**3.** Insecticidal, acaricidal, nematicidal and/or fungicidal compositions, said compositions containing as an active ingredient at least one of pyridazinone derivative of the formula (I):

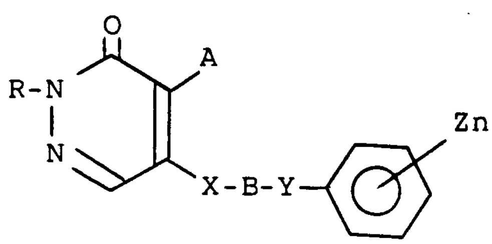

(I)

wherein

R represents tertiary butyl,

A represents chlorine atom,

B represents $-CH_2-CH_2-$,

X represents oxygen atom or sulfur atom,

Y represents oxygen atom,

n is an integer of 1 to 5 and when n is 2 to 5, Z may be same or different,

Z represents halogen atom, straight or branched chain alkyl group having 1 to 10 carbon atoms, straight or branched chain alkoxy group having 1 to 10 carbon atoms, alkylcarbonyl having 2 to 10 carbon atoms,

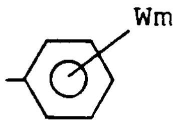 , 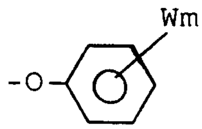 , 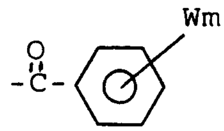 or 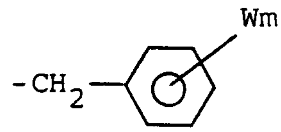

W represents halogen atom, alkyl group having 1 to 4 carbon atoms, alkoxy group having 1 to 4 carbon atoms or nitro group,

m is 0 or an integer of 1 to 2, and when m is 2, W may be same or different.

**4.** A method of controlling various pests on animals and plants which method comprises applying thereto or to locus thereof an effective amount of a compound according to claim 1.

**Revendications**

**1.** Dérivé de pyridazinone de la formule :

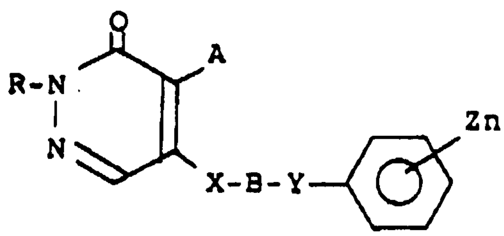

(I)

dans laquelle :

R représente un butyle tertiaire,

A représente un atome de chlore,

a représente $-CH_2-CH_2-$,

x représente un atome d'oxygène ou un atome de soufre,

Y représente un atome d'oxygène,

n est un nombre entier égal à 1 à 5, et, lorsque n est égal à 2 à 5, les Z peuvent être identiques ou différents,

Z représente un atome d'halogène, un groupe alcoyle à chaîne rectiligne ou ramifiée ayant 1 à 10 atomes de carbone, un groupe alcoxy à chaîne rectiligne ou ramifiée ayant 1 à 10 atomes de carbone, un groupe alcoylcarbonyle ayant 2 à 10 atomes de carbone,

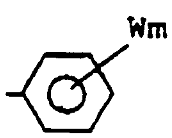 , 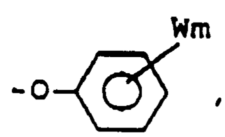 , 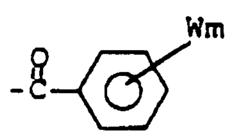 ou 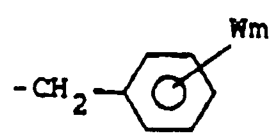

W représente un atome d'halogène, un groupe alcoyle ayant 1 à 4 atomes de carbone, un groupe alcoxy ayant 1 à 4 atomes de carbone ou un groupe nitro, m est égal à 0, 1 ou 2, et, lorsque m est égal à 2, les W peuvent être identiques ou différents.

**2.** Procédé pour préparer un dérivé de pyridazinone de la formule générale (I) :

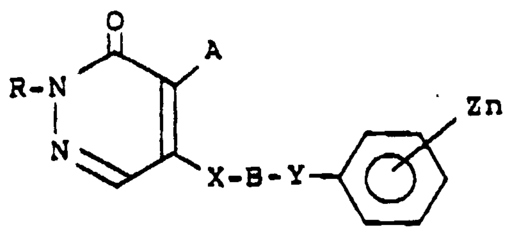

(I)

dans laquelle :

R représente un butyle tertiaire,

A représente un atome de chlore,

B représente $-CH_2-CH_2-$,

X représente un atome d'oxygène ou un atome de soufre,

Y représente un atome d'oxygène,

n est un entier égal à 1 à 5 et, lorsque n est égal à 2 à 5, les Z peuvent être identiques ou différents,

Z représente un atome d'halogène, un groupe alcoyle à chaîne rectiligne ou ramifiée ayant 1 à 10 atomes de carbone, un groupe alcoxy à chaîne rectiligne ou ramifiée ayant 1 à 10 atomes de carbone, un groupe alcoylcarbonyle ayant 2 à 10 atomes de carbone,

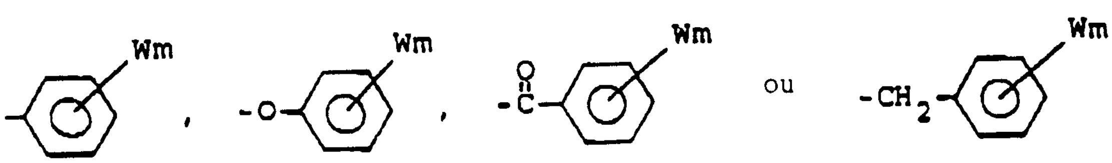

, , ou

W représente un atome d'halogène, un groupe alcoyle ayant 1 à 4 atomes de carbone, un groupe alcoxy ayant 1 à 4 atomes de carbone ou un groupe nitro,

m est égal à 0, 1 ou 2, et, lorsque m = 2, les W peuvent être identiques ou différents, lequel procédé consiste à faire réagir un composé de la formule (II) :

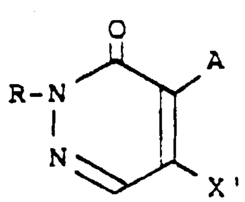

(II)

avec un composé de la formule (III) :

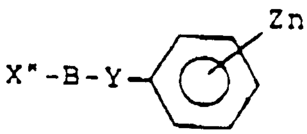

(III)

dans laquelle R, A, B, Y, Z et n ont les mêmes significations que ci-dessus, et X' et X'' représentent un atome d'halogène, -SM ou -OM, dans lequel M désigne un atome d'hydrogène ou un atome de métal alcalin.

**3.** Compositions insecticides, acaricides, nématocides et/ou fongicides, ces compositions contenant comme ingrédient actif au moins un dérivé de pyridazinone de la formule (I) :

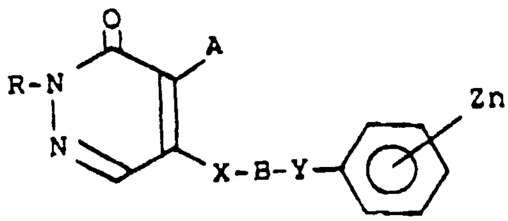

(I)

dans laquelle :

R représente un butyle tertiaire,

A représente un atome de chlore,

B représente $-CH_2-CH_2-$,

X représente un atome d'oxygène ou un atome de soufre,

Y représente un atome d'oxygène,

n est un entier égal à 1 à 5 et, lorsque n est égal à 2 à 5, les Z peuvent être identiques ou différents,

Z représente un atome d'halogène, un groupe alcoyle à chaîne rectiligne ou ramifiée ayant 1 à 10 atomes de carbone, un groupe alcoxy à chaîne rectiligne ou ramifiée ayant 1 à 10 atomes de carbone, un groupe alcoylcarbonyle ayant 2 à 10 atomes de carbone,

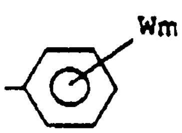 , 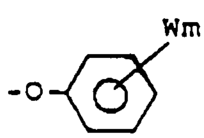 , 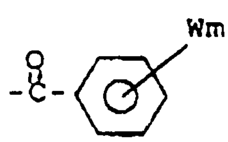 ou 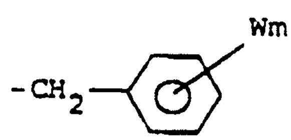

W représente un atome d'halogène, un groupe alcoyle ayant 1 à 4 atomes de carbone, un groupe alcoxy ayant 1 à 4 atomes de carbone ou un groupe nitro,

m est égal à 0, 1 ou 2 et, lorsque m = 2, les W peuvent être identiques ou différents.

**4.** Procédé pour s'attaquer à divers nuisibles sur des animaux et des plantes, lequel procédé consiste à appliquer sur eux ou sur un emplacement sur eux une quantité efficace d'un composé selon la revendication 1.

## Patentansprüche

**1.** Pyridazinonderivat der Formel I

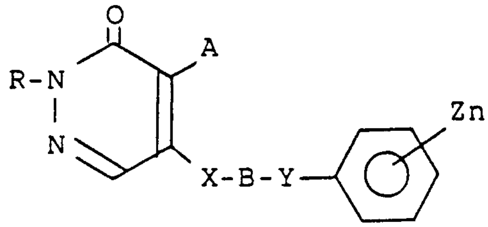

(I),

worin bedeuten:

- R tert.-Butyl;
- A Chlor;
- B $-CH_2-CH_2-$;
- X ein Sauerstoff- oder Schwefelatom;
- Y ein Sauerstoffatom;
- n eine ganze Zahl von 1 bis 5, wobei, wenn n eine ganze Zahl von 2 bis 5 bedeutet, die Gruppen Z gleich oder verschieden sein können;
- Z Halogen, geradkettiges oder verzweigtes Alkyl mit 1 bis 10 Kohlenstoffatomen, geradkettiges oder verzweigtes Alkoxy mit 1 bis 10 Kohlenstoffatomen, Alkylcarbonyl mit 2 bis 10 Kohlenstoffatomen, eine Gruppe

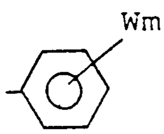 , 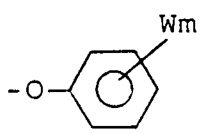 , 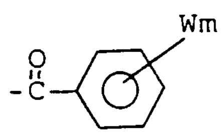 oder 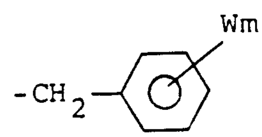

wobei W Halogen, Alkyl mit 1 bis 4 Kohlenstoffatomen, Alkoxy mit 1 bis 4 Kohlenstoffatomen oder Nitro darstellt,

m O oder eine ganze Zahl von 1 bis 2 darstellt und, wenn m 2 ist, die Gruppen W gleich oder verschieden sein können.

**2.** Verfahren zur Herstellung eines Pyridazinonderivates der allgemeinen Formel I

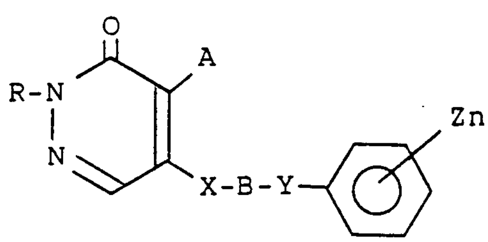

(I),

worin bedeuten:

- R tert.-Butyl;
- A Chlor;
- B $-CH_2-CH_2-$;
- X ein Sauerstoff- oder Schwefelatom;
- Y ein Sauerstoffatom;
- n eine ganze Zahl von 1 bis 5, wobei, wenn n eine ganze Zahl von 2 bis 5 bedeutet, die Gruppen Z gleich oder verschieden sein können;

Z Halogen, geradkettiges oder verzweigtes Alkyl mit 1 bis 10 Kohlenstoffatomen, geradkettiges oder verzweigtes Alkoxy mit 1 bis 10 Kohlenstoffatomen, Alkylcarbonyl mit 2 bis 10 Kohlenstoffatomen, eine Gruppe

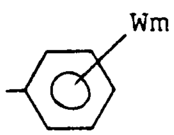 , 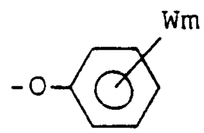 , 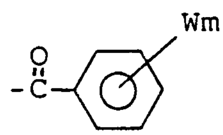 oder 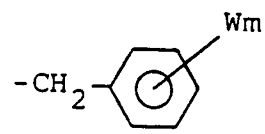

wobei W Halogen, Alkyl mit 1 bis 4 Kohlenstoffatomen, Alkoxy mit 1 bis 4 Kohlenstoffatomen oder Nitro darstellt,

m O oder eine ganze Zahl von 1 bis 2 darstellt, und wenn m 2 ist, die Gruppen W gleich oder verschieden sein können,

dadurch gekennzeichnet, dass man eine Verbindung der Formel II

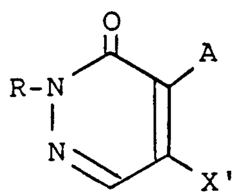 (II)

mit einer Verbindung der Formel III

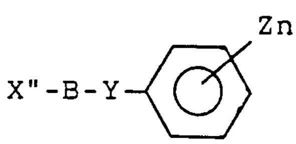 (III),

worin R, A, B, Y, Z und n wie oben definiert sind und X' und X" Halogen, -SM oder -OM bedeuten, wobei M ein Wasserstoffatom oder ein Alkalimetallatom darstellt.

**3.** Insektizide, akarizide, nematizide und/oder fungizide Zubereitungen, welche wenigstens ein Pyridazinonderivat der Formel I

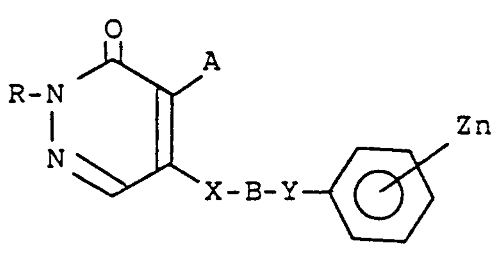 (I),

worin bedeuten:

R tert.-Butyl;
A Chlor;
B $-CH_2-CH_2-$;
X ein Sauerstoff- oder Schwefelatom;
Y ein Sauerstoffatom;
n eine ganze Zahl von 1 bis 5, wobei, wenn n eine ganze Zahl von 2 bis 5 bedeutet, die Gruppen Z gleich oder verschieden sein können;

Z Halogen, geradkettiges oder verzweigtes Alkyl mit 1 bis 10 Kohlenstoffatomen, geradkettiges oder verzweigtes Alkoxy mit 1 bis 10 Kohlenstoffatomen, Alkylcarbonyl mit 2 bis 10 Kohlenstoffatomen, eine Gruppe

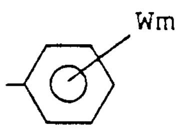 , 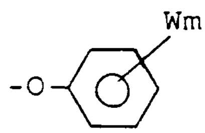 , 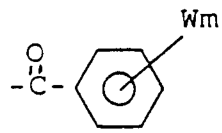 oder 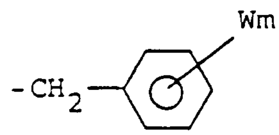

wobei W Halogen, Alkyl mit 1 bis 4 Kohlenstoffatomen, Alkoxy mit 1 bis 4 Kohlenstoffatomen oder Nitro darstellt,

m O oder eine ganze Zahl von 1 bis 2 darstellt und, wenn m 2 ist, die Gruppen W gleich oder verschieden sein können,

als aktive Komponente enthalten.

**4.** Verfahren zur Bekämpfung von verschiedenen Schädlingen auf Tieren und Pflanzen, bei dem man diese oder deren Fundort mit einer wirksamen Menge einer Verbindung nach Anspruch 1 behandelt.